# EUROPEAN PATENT APPLICATION

(11) **EP 2 133 807 A1**
(43) Date of publication of application: **16.12.2009**
(21) Application number: 08721067.0
(22) Date of filing: 29.02.2008
(51) Int. Cl.: G06F 19/00, G06F 17/30

(54) **HOMOLOGOUS SEARCH SYSTEM**

(30) Priority: 02.03.2007 JP 2007052583
(71) Applicant: Research Organization Of Information And Systems, Tokyo 1068569 (JP)
(72) Inventor: The designation of the inventor has not yet been filed
(74) Representative: Jackson, Martin Peter
(86) International application number: PCT/JP2008/053647
(87) International publication number: WO 2008/108297

(57) **Abstract**

A homology retrieval can be performed with higher accuracy than conventional technologies when comparing a query sequence with a target sequence, and retrieving a similar location in the target sequence. The sequence information of a query sequence and a genomic-scale target sequence is acquired, the acquired information is compressingly converted into a compressed query sequence and a compressed target sequence in each of which a homopolymer region including two or more consecutive identical bases is replaced with a single base of the bases, the two sequences are compared, and a refining search is performed for a compressed target partial sequence that matches the compressed query sequence in the compressed target sequence. For the refined compressed candidate sequence and the query sequence, based on the information on the number of consecutive identical bases in the each of the sequences before compression, the number of consecutive bases is compared between the two compressed sequences for each corresponding base, and the degree of similarity indicating homology of the candidate sequence with the query sequence is computed from a degree of match or a degree of mismatch in the number of consecutive bases. By ranking and selecting an arbitrary number of candidate sequences having relatively high homology with the query sequence from this degree of similarity, it is possible to avoid the influence of the number of consecutive identical bases in a homopolymer region, thereby performing a homology retrieval accurately.

## Description

### Technical Field

The present invention relates to a homology retrieval system, a homology retrieval apparatus, a homology retrieval method, and a computer program capable of executing the homology retrieval method on a computer and an electronic medium in which the program is stored.

### Background Art

In the field of life science, the entire genome sequences of many biological species have been revealed in recent years. Also in sequence reading technologies for base sequences, an earlier method of reading a ladder pattern by exposing a silver halide film using autoradiography has been replaced by a method in which a fluorescent label on an electrophoresis lane is excited with laser light and thus automatically read, resulting in a significant advance in automation. Furthermore, a variety of technologies for increasing sensitivity and speed have been introduced, and throughput also has been increased. However, these methods are all based on the same principle called the "Sanger method", and have performance limitations imposed by the constraints on the real physical migration time. Therefore, pyrosequencing technology was newly developed, and has been put into practical use. This technology is based on a principle that is significantly different from the conventional Sanger method, and directly reads fluorescence intensity resulting from a chemical reaction of the elongation of a complementary strand, rather than electrophoresis. By this principle, such pyrosequencing technology has realized a sequencing speed much higher than that can be realized by the Sanger method.

However, the pyrosequencing technology has the following problem with regard to sequencing of a region including a plurality of connected identical bases in a sequence (hereinafter, referred to as a "homopolymer region"). That is, in the pyrosequencing technology, the information on a sequence is only observed as a ratio of fluorescence intensity at the time of measurement that has a dynamic range saturation limit. For this reason, it is difficult to determine the number of identical bases accurately for a homopolymer region where identical bases are successively connected, which results in a problem with the sequencing accuracy. Such a problem with the sequencing accuracy for the homopolymer region has also posed a similar technical limitation on the Sanger method described above. However, due to its high throughput, pyrosequencing technology is more significantly affected by the above-described problem relating to the homopolymer region, as compared with the Sanger method.

On the other hand, for example, for a sequence whose position on a genome is unknown or a sequence whose function, origin or the like is unknown (hereinafter, referred to as a "query sequence"), homology retrievals (similarity retrievals) for retrieving a homologous partial sequence in a sequence of a decoded genome or the like (hereinafter, referred to as a "target sequence") are performed in gene analyses. This homology retrieval technology has undergone little change, in contrast with the above-described dramatic progress in sequencing methods, and the following methods are generally used.

(1) One typical example of the systems that perform a homology retrieval is "BLAST" (Non-patent document 1). This system is widely used and has been established as a standard system for performing a sequence retrieval in the field of life science.
(2) One example of similarity degree retrieval methods in which mismatching of a partial sequence is maximally tolerated by scoring for sequence insertion or deletion is the Smith-Waterman method using dynamic programming (Non-patent document 2). This method is used for implementing a plurality of systems.
(3) In addition, a method has been reported that attempts to solve the problem relating to speeds by incorporating the logic of dynamic programming in (2) above into hardware, and executing metaparallel operations (Patent document 1).
   Non-patent document 1: Altschul S. F., Gish W., Miller W., Myers E. W., and Lipman D. J. (1990) Basic local alignment search tool. J. Mol. Biol. Vol. 215, pp. 403-410.
   Non-patent document 2: Smith TF, Waterman MS. (1981) Comparison of biosequences. Adv. Appl. Math. 2:482-9.
   Patent document 1: JP H07-093370A

### Disclosure of Invention

However, although these homology retrieval methods are performed based on base sequence information determined by base sequencing methods as described above, they cannot avoid the problem with sequencing accuracy for homopolymer regions that is caused by such base sequencing methods. In other words, when a target sequence of a genome or the like that is used for a homology retrieval includes a homopolymer region, there is a problem with accuracy with regard to the number of consecutive identical bases in a homopolymer region that has been determined by a base sequencing method, as described above. However, the above-described homology retrieval methods cannot be said to take such a problem into consideration. Accordingly, there is a problem, for example, in that no result can be extracted due to the influence of the sequence accuracy, or that a result is erroneously extracted even though there is no similarity, for example, even if a partial sequence in a target sequence of a genome or the like actually has high homology with a query sequence.

In BLAST (1) above, when comparing a long query sequence and a target sequence, analysis is performed, taking any mismatch in the number of consecutive identical bases in the query sequence as an insertion or deletion of a base. This enables even sequences that do not completely match to be retrieved in association with each other to some extent.
However, when the query sequence is a short sequence, or when there is a mismatch in the number of consecutive identical bases in the vicinity of both ends of a query sequence, many cases have been observed where such a difference is overrated, so that the entire short query sequence is determined as mismatching, or portions of the above-mentioned ends are determined as mismatching and thus excluded from candidates for a homologous sequence.

The Smith-Waterman method (2) above is less likely to give a mismatch depending on the location of a homopolymer region as compared with BLAST. Furthermore, by finding an alignment showing an optimal base sequence correspondence using a dynamic programming algorithm, it can perform a better search than BLAST. However, a mismatch in the number of consecutive bases in a homopolymer region and a mismatch for another single base are measured on the same scale, so there is still a problem with the reasonableness of homology ranking. Furthermore, the method is disadvantageous in that the retrieval performance is very slow since it requires a computational complexity in the order of the product of the query sequences and target sequences to execute basic dynamic programming. Furthermore, this method is not practical, for example, in the case of handling an exhaustive amount, for example, an extremely large amount exceeding 1,000,000 query sequences resulting from the advance in sequencing methods.

The method (3) above uses the same basic algorithm as that of (2) above, and has the same problems in terms of the operational accuracy. Although the method has been considerably improved in terms of performance, it requires the use of dedicated hardware, and therefore is more expensive than methods using computer software. Furthermore, since the hardware is fixed, the performance specification, including, for example, the reliability, easily becomes obsolete as compared with a system that runs on a general-purpose computer. For this reason, the use of this method is limited to a particular range.

As has been described thus far, all of these homology retrieval methods have problems, for example, with accuracy, performance and cost in the case where there is a mismatch in the number of identical bases in a homopolymer region when comparing a target sequence and a query sequence. For this reason, there is a need for a homology retrieval that is suitable for the case where there is a mismatch in the number of consecutive identical bases in corresponding homopolymer regions of two sequences.

Therefore, it is an object of the present invention to allow a homology retrieval to be performed promptly with higher accuracy than conventional technologies when retrieving a homologous partial sequence in a target sequence for a query sequence, even if there is a difference in the number of consecutive identical bases in corresponding homopolymer regions of two sequences.

In order to achieve the foregoing object, a homology retrieval system according to the present invention is a homology retrieval system that retrieves, using sequence information of a query sequence including a nucleic-acid base sequence, a partial sequence homologous with the query sequence from sequence information of a genomic-scale target sequence including a nucleic-acid base sequence, the system including:
an acquisition unit that acquires the sequence information of the query sequence and the target sequence;
a compressed sequence preparation unit that prepares a compressed query sequence and a compressed target sequence in each of which a homopolymer region including two or more consecutive identical bases is replaced with a single base of the bases respectively for the query sequence and the target sequence that have been acquired;
a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
a consecutive base number preparation unit that prepares information on the number of consecutive identical bases in each of the sequences before compression of the compressed query sequence and the compressed candidate sequence selected by the retrieval unit;
a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in said number of consecutive bases;
a selection unit that ranks and selects an arbitrary number of candidate sequences having relatively high homology with the query sequence, based on a degree of similarity computed by the similarity degree computing unit; and
an output unit that outputs information on the arbitrary number of candidate sequences selected by the selection unit.

A homology retrieval apparatus according to the present invention is a homology retrieval apparatus that retrieves, using sequence information of a query sequence including a nucleic-acid base sequence, a target partial sequence homologous with the query sequence from sequence information of a genomic-scale target sequence including a nucleic-acid base sequence, the apparatus including the homology retrieval system according to the present invention.

A homology retrieval method according to the present invention, is a homology retrieval method for retrieving, using sequence information of a query sequence including a nucleic-acid base sequence, a target partial sequence homologous with the query sequence from sequence information of a genomic-scale target sequence including a nucleic-acid base sequence, the method including:
an acquisition step of acquiring the sequence information of the query sequence and the target sequence;
a compressed sequence preparation step of preparing a compressed query sequence and a compressed target sequence in each of which a homopolymer region including two or more consecutive identical bases is replaced with a single base of the bases respectively for the query sequence and the target sequence that have been acquired;
a retrieval step of comparing the compressed query sequence and the compressed target sequence, and performing a refining search for a compressed target partial sequence that matches the compressed query sequence in the compressed target sequence, and selecting the refined compressed target partial sequence as a compressed sequence of a candidate sequence (compressed candidate sequence);
a consecutive base number preparation step of preparing information on the number of consecutive identical bases in each of the sequences before compression of the compressed query sequence and the compressed candidate sequence selected in the retrieval step;
a similarity degree computing step of comparing, based on the information on the number of consecutive identical bases, the number of consecutive bases between the compressed query sequence and the compressed candidate sequence for each corresponding base, and computing a degree of similarity indicating homology of the candidate sequence with the query sequence from a degree of match or a degree of mismatch in the number of consecutive bases;
a selection step of ranking and selecting an arbitrary number of candidate sequences having relatively high homology with the query sequence, based on a degree of similarity computed in the similarity degree computing step; and
an output step of outputting information on the arbitrary number of candidate sequences selected by the selection step.

A computer program according to the present invention is a computer program capable of executing the homology retrieval method according to the present invention on a computer.

An electronic medium according to the present invention is an electronic medium in which the computer program according to the present invention is stored.

According to the present invention, taking into consideration the problem caused by variations in the number of consecutive identical bases in a homopolymer region that occurs in determining a base sequence, the target sequence and the query sequence are first compared in the form of a compressed sequence (a compressed sequence in which a homopolymer region is replaced with a single base), which is not affected by the number of consecutive identical bases, and the homology between the two sequences is then determined from the number of consecutive bases in a homopolymer region. With conventional methods, variations in the number of consecutive identical bases in a homopolymer region may cause an irrational, inappropriate homology ranking, or variations in the number of consecutive identical bases in a homopolymer region itself may be overlooked. However, the present invention makes it possible to avoid such a problem, thereby enabling selecting a partial sequence of a target sequence that matches a query sequence more accurately. Accordingly, even if an error or a displacement is included in the number of consecutive identical bases in a homopolymer region, due to, for example, the method for determining a base sequence, or the polymorphism of a sequence itself, the present invention can avoid the influence thereof and enables a more accurate homology retrieval. In particular, when the information on a base sequence is determined not only by the conventional Sanger method, but also by a pyrosequencing technology with a high throughput, it is possible to obviate the influence of a low determination accuracy for the number of consecutive identical bases in a homopolymer region. Moreover, since a homology retrieval can be accurately performed in this way, it is also possible to accurately make a determination, for example, as to whether a query sequence and a partial sequence in a target sequence show only a single homology (similarity). Furthermore, since compressed sequences, which do not require taking into consideration the number of consecutive identical bases in a homopolymer region, are compared, and the matched partial sequence of the target sequence is selected, it is also possible to realize cost reductions as compared with conventional technologies due to a further improved data processing capability. Accordingly, the present invention can solve the influence of variations in the number of consecutive identical bases in a homopolymer region, which has been conventionally unsolvable, in the field of homology retrieval (similarity retrieval), and therefore can be considered as a very useful technology particularly in the field of gene analysis.

### Brief Description of Drawings

[FIG. 1] FIG. 1 is a block diagram showing an example of the hardware configuration of a homology retrieval apparatus according to one embodiment of the present invention.
[FIG. 2] FIG. 2 is a diagram schematically showing a homology retrieval system according to another embodiment of the present invention.
[FIG. 3] FIG. 3 is a diagram schematically showing a homology retrieval system according to yet another embodiment of the present invention.
[FIG. 4] FIG. 4 schematically shows compressing conversion and counting of the number of consecutive identical bases according to a further embodiment of the present invention.
[FIG. 5] FIG. 5 is a flowchart illustrating the flow of the processing of a homology retrieval method according to a further embodiment of the present invention.
[FIG. 6] FIG. 6 schematically shows a method for calculating the degree of similarity according to a further embodiment of the present invention.
[FIG. 7] FIG. 7 is a diagram schematically showing an example of a query sequence hash table according to a further embodiment of the present invention.
[FIG. 8] FIG. 8 is a diagram showing the overall configuration of an example of a stand-alone apparatus using a system according to the present invention.
[FIG. 9] FIG. 9 is a diagram showing the overall configuration of an example of a network-utilizing-type apparatus using a system according to the present invention.
[FIG. 10] FIG. 10 is a block diagram showing an example of the configuration of the stand-alone apparatus.
[FIG. 11] FIG. 11 is a block diagram showing an example of the configuration of the network-type apparatus.

### Best Mode for Carrying Out the Invention

In the present invention, "query sequence" is not particularly limited, as long as it is a nucleic-acid base sequence. Examples of the base sequence include genome fragment sequences of various species of organisms and full-length or fragment transcriptome sequences obtained by an oligo-capped method or the like. The length of a query sequence in the present invention is, but is not particularly limited to, for example, 12 to 60 bases, preferably 18 to 25 bases.

In the present invention, "genomic-scale target sequence" includes, but is not particularly limited to, all the nucleic-acid base sequences decoded as a genome, the nucleic-acid base sequence of a whole chromosome, a mutant sequence thereof such as a single nucleotide polymorphism or a haplotype, and a comprehensive collected sequence of transcripts called a transcriptome, which is the nucleic acid replicated from a genome. In addition, as such a genomic-scale target sequence, it is possible to use, for example, sequences registered in various databases (e.g., DDBJ, EMBL, ENSEMBL, GenBank, and UCSC). The target sequence length can be, but is not particularly limited to, for example, the 3 billion base pairs of the human genome, and the present invention is particularly preferably applied to a sequence of one million bases or more.

In the present invention, "homopolymer region" means a region including two or more consecutive (repeating) identical nucleic-acid bases (e.g., adenines, guanines, cytosines or thymines) in a nucleic-acid base sequence.

In the present invention, a "compressed sequence" refers to a sequence resulting from replacing the homopolymer region including two or more consecutive identical bases with a single base of the bases in the sequence information of each of a query sequence and a target sequence. That is, a "compressed sequence" is sequence information indicating a series of nucleic-acid base types for a query sequence and a target sequence. The above-described replacement with a single base of the bases is referred to as a "compressing conversion", a sequence resulting from compressingly converting a query sequence is referred to as a "compressed query sequence", and a sequence resulting from compressingly converting a target sequence is referred to as "compressed target sequence". Further, a "compressed target partial sequence" means a partial sequence in a compressed target sequence that matches the compressed query sequence.

In the present invention, "information on the number of consecutive bases" is sequence information indicating the sequence information of a query sequence and a target sequence as the number of consecutive identical bases that are present, rather than as a series of nucleic-acid base types. When n consecutive identical bases are present, this can be counted as "n". More specifically, for example, this can be counted as "1" when a single identical base is present in a nucleic-acid base sequence, and this can be counted as "2" when two consecutive identical bases are present.

In the present invention, a "target sequence" is a genomic-scale nucleic-acid base sequence, as described above. In a commonly used homology retrieval, a genomic-scale target sequence is usually broken down into partial sequences before performing a retrieval. Specifically, a plurality of partial sequences are generated, for example, by shifting one base at a time from the beginning of a target sequence, and a partial sequence group made up of these partial sequences is used. In the present invention, it is also preferable to perform a retrieval using a target partial sequence group resulting from dividing a target sequence into partial sequences. Therefore, in the present invention, the above-described compressed target sequence may preferably be a compressed target partial sequence group made up of compressed target partial sequences resulting from performing, on a target sequence, the compression processing of replacing a homopolymer region including two or more consecutive identical bases with a single base of the bases and dividing the resulting compressed target sequence after compression into fixed lengths, for example. The fixed length is not limited, and the present invention can be implemented for 1 to 100 bases, for example. Particularly, it is possible to perform a very effective retrieval for a length of 8 to 50 bases. The fixed length may be, for example, the base length of a compressed sequence that is subjected to the below-described hash (hash target base length), and may be a so-called "hash width".

In the present invention, the number of target sequences with which a query sequence is compared is not limited. For example, one query sequence may be compared with one target sequence of interest, or may be compared with two or more target sequences. The number of query sequences is also not limited, and one target sequence is compared with one, or two or more query sequences, for example. A compressed target sequence after compression is divided into fixed lengths as described above to form a compressed target partial sequence group and, thereafter, the below-described hash processing is performed on each compressed target partial sequence, for example. At this time, the fixed length is preferably the same as the length of a compressed query sequence, for example. Then, when a plurality of query sequences are present, it is preferable to generate, from a target sequence after compression, the number of compressed target partial sequence groups corresponding to the number of sequences having varied lengths of the plurality of compressed query sequences, and to perform multiple homology retrievals independently for each of the compressed target partial sequence groups.

In the present invention, the number of candidate sequences selected based on the degree of similarity is not limited, and can be set to an arbitrary number. For example, only a candidate sequence showing the highest homology result may be selected, or the candidate sequences may be ranked in descending order of homology, and the top several sequences may be selected based on that order. When the number of candidate sequences indicating homology does not reach an arbitrary number that has been set, the number of candidate sequences selected may be less than the arbitrary number.

In the following, a homology retrieval system, a homology retrieval apparatus, a homology retrieval method, a computer program capable of executing the homology retrieval method on a computer, and an electronic medium in which the program is stored, according to the present invention, will be described. The homology retrieval method of the invention can be realized, for example, by the homology retrieval system of the invention or the homology retrieval apparatus of the invention, or by executing the computer program of the invention.

According to the present invention, a homology retrieval is performed taking into consideration variations in the number of consecutive identical bases in a homopolymer region as described above, and therefore, it is possible to retrieve, for example, a homologous partial sequence that could not be retrieved by conventional methods, and to avoid the possibility that the existence of homology is erroneously determined as in the case of conventional methods. Furthermore, since compressed sequences that are unaffected by the number of consecutive identical bases in a homopolymer region are compared, the data processing speed can be increased markedly. In terms of the homology retrieval apparatus of the present invention, for example, these effects can be described as follows. When a query sequence is directly subjected to a conventional BLAST retrieval, there may be cases where there is no hit especially if a homopolymer region including consecutive identical bases is present in a plurality of locations in at least one of a query sequence and a target sequence. In order to perform a successful retrieval for all cases by avoiding this, it has been hitherto necessary to generate all combinations within a margin of error for the number of consecutive identical bases in a homopolymer region for the query sequence, and to execute BLAST. However, according to this method, for example, when 1,000,000 simulations are performed by a Monte Carlo method by setting the margin of error for the number of consecutive identical bases to less than twice under the assumption that the probability of the occurrence of 4 bases is uniformly random, it is necessary to retrieve patterns about 135 times for a query sequence length of 25 bases, about 21,500 times for a query sequence length of 50 bases, and about 84,000,000 times for a query sequence length of 100 bases. Then, a retrieval time that is substantially proportional thereto is also required. In contrast, with the present invention, retrieval processing can be performed for any query sequence within a time period in which the score calculation time unique to the present invention (linear to the sequence length) is added to the time corresponding to a single BLAST retrieval, regardless of the query sequence length.

### Homology Retrieval System

As described above, a first homology retrieval system according to the present invention includes:
an acquisition unit that acquires the sequence information of the query sequence and the target sequence;
a compressed sequence preparation unit that prepares a compressed query sequence and a compressed target sequence in each of which a homopolymer region including two or more consecutive identical bases is replaced with a single base of the bases respectively for the query sequence and the target sequence that have been acquired;
a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
a consecutive base number preparation unit that prepares information on the number of consecutive identical bases in each of the sequences before compression of the compressed query sequence and the compressed candidate sequence selected by the retrieval unit;
a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in the number of consecutive bases;
a selection unit that ranks and selects an arbitrary number of candidate sequences having relatively high homology with the query sequence, based on a degree of similarity computed by the similarity degree computing unit; and
an output unit that outputs information on the arbitrary number of candidate sequences selected by the selection unit.

As described above, the system according to the present invention is characterized by retrieving homology by comparing compressed sequences and the number of consecutive identical bases for a query sequence and a target sequence, and there is no particular limitation with respect to conditions and configurations other than this.

Examples of the sequence information acquired by the acquisition unit include sequence information before compression. Alternatively or additionally to the sequence before compression, for example, information on a compressed sequence, the number of consecutive identical bases and the like may be included as the sequence information. When a compressed sequence is acquired as the sequence information by the acquisition unit, the acquisition unit and the compressed sequence preparation unit that prepares a compressed sequence can be considered as the same unit, for example. When information on the number of consecutive identical bases is acquired as the sequence information by the acquisition unit, the acquisition unit and the consecutive base number preparation unit that prepares information on the number of consecutive identical bases can be considered as the same unit, for example. Further, the sequence information of a compressed sequence and the number of consecutive identical bases may be acquired only for one of a query sequence and a target sequence, and the sequence information before compression may be acquired for the other sequence. In this case, information on a compressed sequence and the number of consecutive identical bases may be acquired from the sequence information before compression only for the other sequence, using a unit as described below.

In the system of the present invention, the acquisition unit of sequence information is not particularly limited, and examples thereof include an input unit that inputs sequence information of a target sequence and/or a query sequence. In this case, it is preferable that the system of the invention further includes, for example, a storage unit that stores sequence information of a query sequence that has been input (query sequence storage unit) and a storage unit that stores sequence information of a target sequence that has been input (target sequence storage unit). As described above, examples of the sequence information of a query sequence include, in addition to a sequence before compression, information on a compressed sequence, and the number, the origin and the like of consecutive identical bases. As described above, examples of the sequence information of a target sequence include, in addition to a sequence before compression, a compressed sequence, the number and the origin of consecutive identical bases, the presence or absence of the functions of various regions in a target sequence, and the details of such functions.

For example, an input unit that inputs the sequence information of a query sequence and a storage unit in which the sequence information of a target sequence is stored (target sequence storage unit) may be included as the acquisition unit of sequence information. A target sequence that is to be retrieved can be called from the target sequence storage unit, for example, by specifying a desired sequence in the sequences stored in the target sequence storage unit. Further, it is preferable to further include a storage unit that stores the sequence information of a query sequence that has been input (query sequence storage unit).

As described above, examples of the target sequence storage unit include a database in which the sequence information of a target sequence is stored (target sequence database). There is no limitation on the number of the target sequences stored. Examples of such a target sequence database include known databases in which the nucleic-acid base sequences of various genomes and chromosomes are stored. The target sequence database is not limited, and examples thereof include a database connected via a communication network and a removable recording medium in which a target sequence is stored. The database may be stored in a storage unit (storage device) of a computer system.

In addition to the sequence information before compression, for example, the compressed target sequence, the number and the origin of consecutive identical bases in the target sequence, the presence or absence of the functions of various regions in the target sequence, and the details of such functions and the like may be further stored in the target sequence storage unit as the information on a target sequence. By storing information on a compressed target sequence, the number of consecutive identical bases and the like in the storage unit in this way, the necessary information such as a compressed sequence and the number of consecutive identical bases can be called from the target sequence storage unit when further retrieving homology with another query sequence, for example. This makes it possible to save the time and effort for performing the below-described compressing conversion and counting of the number of identical bases again, thereby further enhancing the retrieval capability of the system. Furthermore, in the case of performing a retrieval for a target sequence that is not stored in the target sequence storage unit in the system of the invention, it is preferable that the target sequence information that has been input by an input unit is added to the target sequence storage unit.

The acquisition unit of sequence information may include, for example, an input unit that inputs the sequence information of a target sequence, and a storage unit in which the sequence information of a query sequence is stored (query sequence storage unit). Examples of the storage unit include a database in which the sequence information of a query sequence is stored (query sequence database). The query sequence that is to be retrieved can be called from the query sequence storage unit, for example, by specifying a desired sequence in the sequences stored in the query sequence storage unit. The specification may also be performed, for example, by inputting information associated with a sequence that is to be specified (e. g., a sequence ID) by the input unit. Further, it is preferable to further include a storage unit that stores the sequence information of a target sequence that has been input (target sequence storage unit).

In addition to the sequence information before compression, for example, the compressed query sequence, the number, the origin and the like of consecutive identical bases in a query sequence may be further stored in the query sequence storage unit as the information on a query sequence. By storing information on a compressed query sequence, the number of consecutive identical bases and the like in this way, the necessary information such as a compressed sequence and the number of consecutive identical bases can be called from the query sequence storage unit when retrieving homology with another target sequence, for example. This makes it possible to save the time and effort for performing the below-described compressing conversion and counting of the number of identical bases again, thereby further enhancing the retrieval capability of the system. Furthermore, in the case of performing a retrieval for a query sequence that is not stored in the query sequence storage unit in the system of the invention, it is preferable that sequence information of the query sequence that has been input by the input unit is added to the query sequence storage unit.

The acquisition unit of sequence information may include, for example, a storage unit in which the query sequence is stored (query sequence storage unit) and a storage unit in which the sequence information of the target sequence is stored (target sequence storage unit). For example, in the above-described various storage unit, sequence information of each sequence may be stored in advance, and sequence information that has been input by the input unit further may be stored. Then, a query sequence and a target sequence that are to be retrieved can be called from the various storage unit, for example, by specifying a desired sequence that is to be retrieved in the sequences stored in the respective storage unit.

As described above, it is preferable that new information is added to the various storage unit (databases), and therefore, it is preferable that the system of the invention further includes information updating unit (database updating unit) for adding information.

The compressed query sequence and the compressed target sequence each may be acquired by compressing conversion performed in the system, or may be input by the input unit, or may be called from the above-described various storage unit if they have been stored in advance as the sequence information. That is, examples of the compressed sequence preparation unit in the system of the present invention include a unit that performs compressing conversion into a compressed query sequence and/or a compressed target sequence based on the sequence information of a query sequence and/or a target sequence that have been acquired (compressing conversion unit). Alternatively, when the compressed sequences are respectively stored in the query sequence storage unit and/or the target sequence storage unit, the compressed sequence preparation unit may be a query sequence storage unit in which a compressed query sequence is stored and/or a target sequence storage unit in which a compressed target sequence is stored. In the latter case, the compressed sequences can be called from the respective storage unit by specifying a desired sequence that is to be retrieved.

The numbers of consecutive identical bases in the query sequence and the target sequence each may be acquired by counting processing performed in the system, or may be input by the input unit, or may be called from the above-described various storage unit if they have been stored in advance. That is, examples of the consecutive base number preparation unit in the system of the present invention include a counting unit (consecutive base number counting (computing) unit) that counts (computes) the number of consecutive identical bases in each of the sequences before compression of a query sequence and/or a target sequence based on the sequence information of the acquired query sequence and/or target sequence. Alternatively, when the information on the numbers of consecutive bases is respectively stored in the query sequence storage unit and/or the target sequence storage unit, the consecutive base number preparation unit may be a query sequence storage unit in which information on a query sequence is stored and/or a target sequence storage unit in which information on a target sequence is stored. In the latter case, information on the numbers of consecutive bases can be called from the respective storage unit by specifying a desired sequence that is to be retrieved.

In the present invention, the above-described retrieval unit is not particularly limited, and examples thereof include a hash retrieval unit, a binary tree retrieval unit, and a B-tree retrieval unit. For example, the hash retrieval unit uses the compressed query sequence and compressed target partial sequences of the compressed target partial sequence group as a key, and performs a refining search for the compressed target partial sequence that matches the compressed query sequence by performing a hash retrieval using the same hash function. In the present invention, it is an important factor that, when performing a hash retrieval, the compressed query sequence and the compressed target partial sequences of the compressed target partial sequence group, for example, are used as a key (element), rather than using, for example, an uncompressed query sequence and target partial sequences of an uncompressed partial sequence group as a key (element), and a hash retrieval is performed using the same hash function. Except for this factor, that is, for example, the setting of a hash function and the hash retrieval method itself may be based on any conventionally known method.

In the case of performing a hash retrieval using the homology retrieval system according to the present invention, it is preferable to further include a target sequence hash table generating unit that uses compressed target partial sequences of the compressed target partial sequence group as a key, and generates a target sequence hash table using the same hash function. In this case, the retrieval unit is a hash retrieval unit that may, for example, use the compressed query sequence as a key, and perform a refining search for a compressed target partial sequence that matches the compressed query sequence by performing a hash retrieval with the target sequence hash table generated by the target sequence hash table generating unit, using the same hash function as that used by the target sequence hash table generating unit. By generating a target sequence hash table in this manner, it is possible to perform a hash retrieval by accessing this table. Accordingly, even if the target sequence is a large nucleic-acid base sequence of genomic-scale, it is possible to reduce the calculation time further. It should be noted that the hash table may be generated in the same manner as in any conventionally known method, except for using compressed sequences as a key (the same applies to the following).

In the case of performing a hash retrieval, it is preferable to further include a query sequence hash table generating unit that uses two or more pieces of the compressed query sequences as a key, and generates a query sequence hash table using the same hash function. In this case, the retrieval unit is a hash retrieval unit that can, for example, use compressed target partial sequences of the compressed target partial sequence group as a key, and perform a refining search for compressed target partial sequences that match the compressed query sequences by performing a hash retrieval with the query sequence hash table generated by the query sequence hash table generating unit, using the same hash function as that used by the query sequence hash table generating unit. By generating a query sequence hash table in this way, it is possible to perform a hash retrieval, for example, by sequentially accessing the query sequences, so that it is possible to reduce the calculation time further even if there is a large number of query sequences.

Although various hash tables may be generated within the system in the present invention in this way, it is also possible to adopt a configuration in which a hash table that has been generated outside the system in advance is input. In addition, the target sequence hash table may be stored in the various databases described above. The hash table will be described later.

Preferably, the similarity degree computing unit uses a degree of mismatch in the number of consecutive bases for each corresponding base as a penalty score, and computes a degree of similarity by adding the penalty scores for each corresponding base. However, it is preferable to exclude a mismatch where the number of consecutive identical bases in an upstream terminal base or a downstream terminal base of the compressed query sequence before compression is less than the number of consecutive identical bases in an upstream terminal base or a downstream terminal base of the compressed candidate sequence before compression. The computation of the degree of similarity will be described later.

Preferably, the homology retrieval system of the present invention further includes a storage unit in which information on the query sequence and the arbitrary number of candidate sequences selected by the selection unit is stored. It is preferable to sequentially store the query sequence and information of a candidate sequence showing homology therewith in this way. This storage unit may be, for example, the above-described query sequence storage unit or target sequence storage unit. In the former case, it is preferable to store information on the candidate sequence in association with the query sequence, and in the latter case, it is preferable to store information on the query sequence in association with the target sequence. Although the number of candidate sequences stored is not limited, it is preferably set to a desired number (an arbitrary number). Then, when the number of the stored candidate sequences has reached the arbitrary number, it is preferable to compare the degree of similarity between the stored candidate sequences and a new candidate sequence to rank the candidate sequences in descending order of homology again, and to store an arbitrary number of candidate sequence. Therefore, in such a case, when a degree of similarity of a new candidate sequence to the query sequence has been computed by the similarity degree computing unit, it is preferable that the selection unit re-selects, based on the new degree of similarity and the degree of similarity to the query sequence of the arbitrary number of candidate sequences stored by the storage unit, an arbitrary number of candidate sequences from the above-mentioned candidate sequences. Then, when a plurality of candidate sequences that are homologous with the query sequence are present, it is preferable that information of the query sequence and the candidate sequences is stored. When a new candidate sequence is further retrieved, it is preferable that an arbitrary number of candidate sequences are selected again from the plurality of candidate sequences. This enables selecting a particularly homologous sequence, for example, even if many candidate sequences that are homologous with the query sequence are retrieved.

In the case of performing a retrieval for determining whether a query sequence is homologous specifically only with a certain partial sequence of a target sequence using the homology retrieval system of the present invention, it is preferable to further include a hash table updating unit that updates data of the query sequence hash table. The hash table updating unit has the function of deleting, when two or more candidate sequences having the same degree of similarity that show the highest homology are selected for a single query sequence by the selection unit, the query sequence and the two or more candidate sequences selected therefor from the data of the query sequence hash table. If two or more candidate sequences having the same degree of similarity that show the highest homology are selected for the query sequence, it can be determined that this query sequence is not homologous specifically with these candidate sequences. Accordingly, to retrieve another query sequence that is homologous specifically with these partial sequences of the target sequence, it is preferable to delete the data of the query sequence that did not show specificity from the hash table, as described above. This can further improve the retrieval efficiency.

### Network-type Homology Retrieval System

A second homology retrieval system according to the present invention may be a system including a terminal and a server that are shown below, or in other words, a network-type homology retrieval system. It should be noted that this system is the same as the above-described homology retrieval system, unless otherwise indicated.

That is, a homology retrieval system according to the present invention is a homology retrieval system that retrieves, using sequence information of a query sequence including a nucleic-acid base sequence, a target partial sequence homologous with the query sequence from sequence information of a genomic-scale target sequence including a nucleic-acid base sequence, the system including:
a terminal and a server,
the terminal and the server being connectable via a communication network outside the system,
the terminal including:
   a terminal-side transmission unit that transmits information within the terminal to the server via the communication network;
   a terminal-side receiving unit that receives information transmitted from the server via the communication network;
   a display unit that displays information within the terminal; and
   an acquisition unit that acquires the sequence information of the query sequence,
   the server including:
      a server-side transmission unit that transmits information within the server to the terminal via the communication network;
      a server-side receiving unit that receives information transmitted from the terminal via the communication network;
      a target sequence database in which a target sequence is stored,
      a compressed sequence preparation unit that prepares a compressed query sequence and a compressed target sequence in each of which a homopolymer region including two or more consecutive identical bases is replaced with a single base of the bases respectively for the target sequence in the target sequence database and the query sequence received by the server-side receiving unit;
      a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
      a consecutive base number preparation unit that prepares information on the number of consecutive identical bases in each of the sequences before compression of the compressed query sequence and the compressed candidate sequence selected by the retrieval unit;
      a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in the number of consecutive bases; and
      a selection unit that ranks and selects an arbitrary number of candidate sequences having relatively high homology with the query sequence, based on a degree of similarity computed by the similarity degree computing unit,
wherein information on the query sequence is transmitted from the terminal-side transmission unit to the server-side receiving unit, information on the arbitrary number of candidate sequences selected by the selection unit of the server is transmitted from the server-side transmission unit to the terminal-side receiving unit, and the information on the arbitrary number of candidate sequences that has been received is displayed by the display unit in the terminal.

The various unit in the second homology retrieval system are the same as the above-described first homology retrieval system, for example. For example, the acquisition unit of sequence information may be an input unit as with the above-described first homology retrieval system, or may be a storage unit in which a query sequence is stored.

### Server

A server according to the present invention is a server used for the second homology retrieval system of the invention. It should be noted that the second homology retrieval system is the same as the above-described first homology retrieval system, unless otherwise indicated.

A server according to the present invention includes:
a server-side transmission unit that transmits information within the server to a terminal via the communication network;
a server-side receiving unit that receives information transmitted from the terminal via the communication network;
a target sequence database in which a target sequence is stored,
a compressed sequence preparation unit that prepares a compressed query sequence and a compressed target sequence in each of which a homopolymer region including two or more consecutive identical bases is replaced with a single base of the bases respectively for the target sequence in the target sequence database and the query sequence received by the server-side receiving unit;
a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
a consecutive base number preparation unit that prepares information on the number of consecutive identical bases in each of the sequences before compression of the compressed query sequence and the compressed candidate sequence selected by the retrieval unit;
a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in the number of consecutive bases; and
a selection unit that ranks and selects an arbitrary number of candidate sequences having relatively high homology with the query sequence, based on a degree of similarity computed by the similarity degree computing unit. It should be noted that various unit in the server are the same as the unit in the above-described system.

### Terminal

A terminal according to the present invention is a terminal used for the second homology retrieval system of the invention. It should be noted that the second homology retrieval system is the same as the above-described first homology retrieval system, unless otherwise indicated.

A terminal according to the present invention includes:
a terminal-side transmission unit that transmits information within the terminal to the server via the communication network;
a terminal-side receiving unit that receives information transmitted from the server via the communication network;
a display unit that displays information within the terminal; and
an acquisition unit that acquires sequence information of the query sequence,
wherein information on the query sequence is transmitted from the terminal-side transmission unit to the server-side receiving unit, information on the arbitrary number of candidate sequences selected by the selection unit of the server is transmitted from the server-side transmission unit to the terminal-side receiving unit, and the information on the arbitrary number of candidate sequences that has been received is displayed by the display unit in the terminal.

### Homology Retrieval Apparatus

A homology retrieval apparatus according to the present invention is a homology retrieval apparatus that retrieves, using sequence information of a query sequence including a nucleic-acid base sequence, a partial sequence homologous with the query sequence from sequence information of a genomic-scale target sequence including a nucleic-acid base sequence, the apparatus including the homology retrieval system according to the present invention. The homology retrieval apparatus includes, for example, an acquisition unit that acquires the sequence information of the query sequence and the target sequence;
a compressed sequence preparation unit that prepares a compressed query sequence and a compressed target sequence in each of which a homopolymer region including two or more consecutive identical bases is replaced with a single base of the bases respectively for the query sequence and the target sequence that have been acquired;
a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
a consecutive base number preparation unit that prepares information on the number of consecutive identical bases in each of the sequences before compression of the compressed query sequence and the compressed candidate sequence selected by the retrieval unit;
a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in the number of consecutive bases;
a selection unit that ranks and selects an arbitrary number of candidate sequences having relatively high homology with the query sequence, based on a degree of similarity computed by the similarity degree computing unit; and
an output unit that outputs information on the arbitrary number of candidate sequences selected by the selection unit. Further, as with the above-described system, it may include a target sequence storage unit that stores or have stored sequence information of a target sequence, a query sequence storage unit that stores or has stored sequence information of a query sequence, an input unit of sequence information, an information updating unit for updating the information in the various storage units, and the like.

### Homology Retrieval Method

A homology retrieval method according to the present invention is a homology retrieval method for retrieving, using sequence information of a query sequence including a nucleic-acid base sequence, a partial sequence homologous with the query sequence from sequence information of a genomic-scale target sequence including a nucleic-acid base sequence, the method including:
an acquisition step of acquiring the sequence information of the query sequence and the target sequence;
a compressed sequence preparation step of preparing a compressed query sequence and a compressed target sequence in each of which a homopolymer region including two or more consecutive identical bases is replaced with a single base of the bases respectively for the query sequence and the target sequence that have been acquired;
a retrieval step of comparing the compressed query sequence and the compressed target sequence, and performing a refining search for a compressed target partial sequence that matches the compressed query sequence in the compressed target sequence, and selecting the refined compressed target partial sequence as a compressed sequence of a candidate sequence (compressed candidate sequence);
a consecutive base number preparation step of preparing information on the number of consecutive identical bases in each of the sequences before compression of the compressed query sequence and the compressed candidate sequence selected in the retrieval step;
a similarity degree computing step of comparing, based on the information on the number of consecutive identical bases, the number of consecutive bases between the compressed query sequence and the compressed candidate sequence for each corresponding base, and computing a degree of similarity indicating homology of the candidate sequence with the query sequence from a degree of match or a degree of mismatch in the number of consecutive bases;
a selection step of ranking and selecting an arbitrary number of candidate sequences having relatively high homology with the query sequence, based on a degree of similarity computed in the similarity degree computing step; and
an output step of outputting information on the arbitrary number of candidate sequences selected by the selection step.

The homology retrieval method of the present invention is characterized by retrieving homology by comparing a compressed sequence and the numbers of consecutive identical bases between a query sequence and a target sequence, as described above, and there is no particular limitation on conditions and configurations other than that.

The sequence information acquired by the acquisition step is the same as the sequence information acquired by the acquisition step of the above-described homology retrieval system, and examples thereof include sequence information before compression of a target sequence and a query sequence. Alternatively or additionally to the sequence before compression, information of a compressed sequence, the number of consecutive identical bases and the like may be included as the sequence information. When a compressed sequence is acquired as the sequence information by the acquisition step, the acquisition step and the compressed sequence preparation step of preparing a compressed sequence can be considered as the same step, for example. When information on the number of consecutive identical bases is acquired as the sequence information by the acquisition step, the acquisition step and the consecutive base number preparation step of preparing information on the number of consecutive identical bases can be considered as the same step, for example. Further, the sequence information of a compressed sequence and the number of consecutive identical bases or the like may be acquired only for one of a query sequence and a target sequence, and the sequence information before compression may be acquired for the other sequence.

The acquisition step of sequence information may be, for example, an input step of inputting sequence information. It may also be a calling step of calling sequence information from a storage unit (e.g., a database) in which the sequence information is stored. Both a target sequence and a query sequence may be input by the input step, or one of them may be input by the input step. Further, both a target sequence and a query sequence may be called from the storage unit by the calling step, or one of them may be called from the storage unit by the calling step, and the other may be input by the input step. In the present invention, for example, the acquisition step may be configured as an input step of inputting both the query sequence and the target sequence, configured to include an input step of inputting the query sequence and a calling step of calling sequence information of the target sequence from a target sequence storage unit in which the target sequence is stored, configured to include an input step of inputting the target sequence and a calling step of calling sequence information of the query sequence from a query sequence storage unit in which the query sequence is stored, or configured as a calling step of calling a query sequence and a target sequence respectively from a query sequence storage unit in which the query sequence is stored and a target sequence storage unit in which the target sequence is stored.

Preferably, the homology retrieval method according to the present invention further includes a query sequence storing step of storing sequence information of a query sequence and/or a target sequence storing step of storing sequence information of a target sequence. Preferably, the sequence information is stored, for example, in a query sequence storage unit and a target sequence storage unit as described above. Examples of the stored sequence information include stored information that has been input by the input step, sequence information of a compressed sequence that has been compressingly converted by a compressing conversion step described below and information on the number of consecutive identical bases that has been prepared by the consecutive base number preparation step.

In the homology retrieval method according to the present invention, the numbers of consecutive identical bases in a query sequence and a target sequence may be each obtained based on sequence information that has been acquired, or, when they have been stored in advance as sequence information in a storage unit as described above, they may be called from the above-described various storage unit. That is, in the homology retrieval method of the invention, the consecutive base number preparation step may be, for example, a counting step (consecutive base number counting (computing) step) of counting (computing) the number of consecutive identical bases in each of the sequences before compression of a query sequence and/or a target sequence based on the acquired sequence information of the query sequence and/or the target sequence. Alternatively, when the information on the numbers of consecutive bases is stored in the query sequence storage unit and/or the target sequence storage unit, the consecutive base number preparation step may be a step of calling that information from a query sequence storage unit in which information on the query sequence is stored and/or a target sequence storage unit in which information on the target sequence is stored. In the latter case, the information on the numbers of consecutive bases can be called from the respective storage unit by specifying a desired sequence that is to be retrieved.

In the homology retrieval method according to the present invention, the compressed query sequence and the compressed target sequence may be each obtained based on sequence information that has been acquired, or, when they have been stored in advance as sequence information in a storage unit as described above, they may be called from the above-described various storage unit. That is, in the homology retrieval method of the invention, the compressed sequence preparation step may be, for example, a compressing conversion step of performing compressing conversion into a compressed query sequence and/or a compressed target sequence based on the acquired sequence information of the query sequence and/or the target sequence. Alternatively, when the compressed sequences are respectively stored in the above-described query sequence storage unit and/or target sequence storage unit, the compressed sequence preparation step may be a step of calling the compressed sequences from a query sequence storage unit in which the compressed query sequence is stored and/or a target sequence storage unit in which the compressed target sequence is stored. In the latter case, the compressed sequences can be called from the respective storage unit by specifying a desired sequence that is to be retrieved.

The above-described retrieval step is not particularly limited, and examples thereof include a hash retrieval step, a binary tree retrieval step, and a B-tree retrieval step. For example, the hash retrieval step uses the compressed query sequence and compressed target partial sequences of the compressed target partial sequence group as a key, and performs a refining search for the compressed target partial sequence that matches the compressed query sequence by performing a hash retrieval using the same hash function.

In the case of performing a hash retrieval in the homology retrieval method according to the present invention, it is preferable to further include a target sequence hash table generating step of using compressed target partial sequences of the compressed target partial sequence group as a key, and generating a target sequence hash table using the same hash function. In this case, the retrieval step is a hash retrieval step that uses the compressed query sequence as a key, and perform a refining search for the compressed target partial sequence that matches the compressed query sequence by performing a hash retrieval with the target sequence hash table generated by the target sequence hash table generating step, using the same hash function as that used by the target sequence hash table generating step, for example.

In the case of performing a hash retrieval, it is preferable to further include a query sequence hash table generating step of using two or more pieces of the compressed query sequences as a key, and generating a query sequence hash table using the same hash function. In this case, the retrieval step is a hash retrieval step of, for example, using compressed target partial sequences of the compressed target partial sequence group as a key, and performing a refining search for the compressed target partial sequences that match the compressed query sequences by performing a hash retrieval with the query sequence hash table generated by the query sequence hash table generating step, using the same hash function as that used by the query sequence hash table generating step.

Preferably, the similarity degree computing step uses a degree of mismatch in the number of consecutive bases for each corresponding base as a penalty score, and computes a degree of similarity by adding the penalty scores for each corresponding base. However, it is preferable to exclude a mismatch where the number of consecutive bases in an upstream terminal base or a downstream terminal base of the compressed query sequence before compression is less than the number of consecutive bases in an upstream terminal base or a downstream terminal base of the compressed candidate sequence before compression.

In the homology retrieval method according to the present invention, it is preferable that, when a degree of similarity of a new candidate sequence to the query sequence has been computed in the similarity degree computing step, the selection step re-selects, based on the new degree of similarity and the degree of similarity to the query sequence of the arbitrary number of candidate sequences previously selected by the selection step, an arbitrary number of candidate sequences from said candidate sequences. In such a case, it is preferable to further include, for example, a storing step of storing information of the query sequence and the arbitrary number of candidate sequences selected by the selection step. By storing, for example, the degree of similarity of the selected arbitrary number of candidate sequences to the query sequence in a storage unit as described above in the storing step, the candidate sequence can be readily selected again between the previous degree of similarity and a new degree of similarity.

In the case of performing a retrieval for determining whether a query sequence is homologous specifically only with a certain partial sequence of a target sequence using the homology retrieval method of the present invention, it is preferable to further include a hash table updating step of updating data of the query sequence hash table. For example, the hash table updating step deletes, when two or more candidate sequences having the same degree of similarity that show the highest homology are selected for a single query sequence by the selection step, the query sequence and the two or more candidate sequences selected therefor from the data of the query sequence hash table.

### Computer Program

A computer program according to the present invention is a computer program capable of executing the homology retrieval method according to the present invention on a computer.

### Electronic Medium

An electronic medium according to the present invention is an electronic medium in which the computer program according to the present invention is stored. The electronic medium is a computer readable medium, and may be a recording medium, for example.

### Embodiment 1

### Hardware Configuration

The hardware configuration of a homology retrieval apparatus according to the present invention will be described schematically. It should be noted that the following configuration is merely an example, and the present invention is not limited thereto.

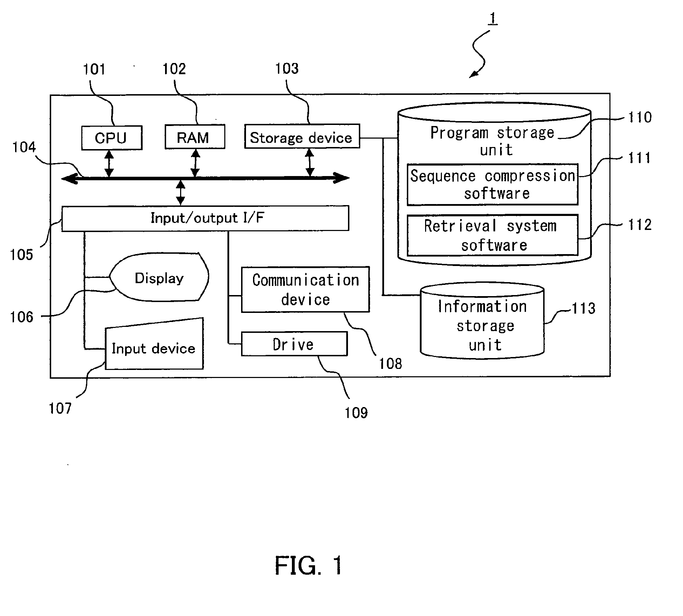

FIG. 1 is a block diagram showing an example of the hardware configuration of a homology retrieval apparatus according to the present invention. In FIG. 1, a homology retrieval apparatus 1 includes a CPU 101, a RAM 102, a storage unit (storage device) 103, an input/output I/F (interface) 105, a display unit (display) 106, an input unit (input device) 107, a communication device 108, and a drive 109. The RAM 102, the storage device 103 and the input/output I/F (interface) 105 are connected to the CPU 101 by a communication bus 104. The display 106, the input device 107, the communication device 108 and the drive 109 are connected to the input/output I/F (interface) 105.

The CPU 101 performs overall control of the homology retrieval apparatus 1. The RAM 102 is a computer main memory, and is a work memory of the CPU 101. The storage device 103 is a ROM, an HDD, or an HD, for example. A ROM is a read-only memory, and stores an operating program. An HDD controls reading and writing of data to and from an HD under the control of the CPU 101, and an HD stores data that has been written under the control of the HDD. The drive 109 is a drive for a removable recording medium, and controls the reading or writing of data to the removable recording medium under the control of the CPU 101. As the removable recording medium, it is possible to use, for example, an FD, a CD-ROM (a CD-R, a CD-RW), an MO, a DVD and a memory card, and these recording media store data that has been written under the control of the drive 109. Ordinarily, the RAM 102 serves as a main storage device, and an external recording medium such as a ROM, a HD and a FD serves as an auxiliary storage device. In the present invention, the CPU 101 executes, for example, a computer program according to the present invention and other programs, and performs reading and writing of various pieces of information. The homology retrieval apparatus 1 shown in FIG. 1 has an exemplary form in which a program storage unit 110 that stores various pieces of software (sequence compression software 111 and retrieval system software 112) and an information storage unit 113 that stores information. These storage units may be provided in a fixed area secured in the above-described auxiliary storage device, for example. In FIG. 1, the program storage unit 110 and the information storage unit 113 are shown as storage areas secured in the storage device 103. For example, these pieces of software are called onto the RAM 102 by the CPU 101 and executed in conjunction with an OS (operation system) and, thereby, their functions are realized. The sequence compression software 111 is a program for compressingly converting a query sequence and a target sequence, and the retrieval system software 112 is a program that executes processes of the present invention other than compressing conversion. In addition, these programs may be a single piece of software implemented as a program according to the present invention.

The display 106 displays various pieces of information such as a document, and examples thereof include an LED display and a liquid crystal display. The I/F (interface) 105 is connected to an external network such as a LAN and the internet via the communication device 108, and connected to another server or information processing apparatus via the external network. In the present invention, the I/F (interface) 105 is connected to an external database (DB) that includes the nucleic-acid base sequence data of genomes or genes, for example. The I/F (interface) 105 serves as an interface between the above-mentioned network and the interior of the apparatus, and controls data input/output to/from another server or the like. The communication device 108 is a modem, for example. Examples of the input device 107 include a keyboard and a mouse, with which the input of characters, numbers, or various instructions, the movement of a cursor, and the like are performed. In addition to these constituent units, it is possible to include a scanner, a printer or the like, for example. The scanner can, for example, optically read image information such as a document, and capture the information as image data. It is also possible to include a printer, which prints out various pieces of information.

### Embodiment 2

An example of each of the configurations of a first homology retrieval system and a second network-type homology retrieval system according to the present invention will be described.

### Configuration Example of First System

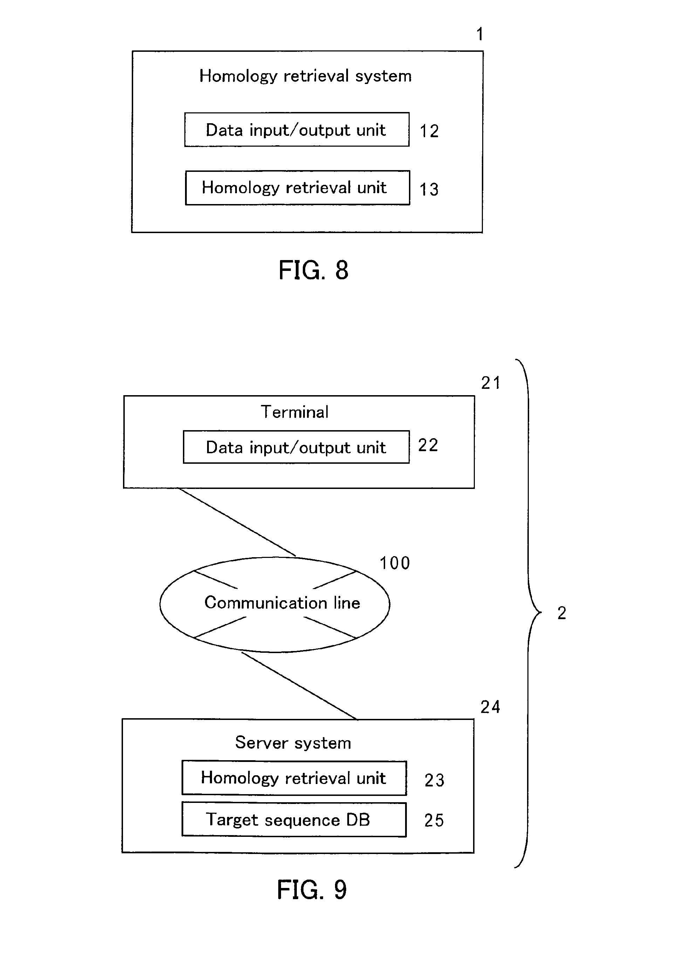

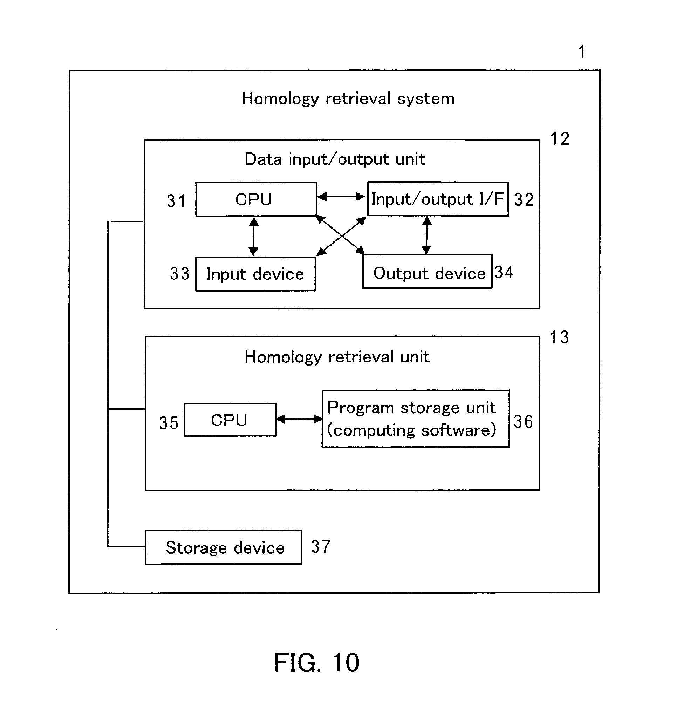

FIG. 8 shows a diagram of an overall configuration of a stand-alone system, which is an example of the configuration of a system according to the present invention. The system shown in FIG. 8 includes a homology retrieval system 1 according to the present invention, and the homology retrieval system 1 includes a data input/output unit 12 and a homology retrieval unit 13. The homology retrieval unit 13 includes, for example, a sequence information acquisition unit, a compressed sequence preparation unit (e.g., a compressing conversion unit) that prepares a compressed sequence, a compressed candidate sequence retrieval unit, a consecutive identical base number preparation unit (e.g., a consecutive base number counting unit), a similarity degree computing unit, and a candidate sequence selection unit. FIG. 10 shows an example of the hardware configuration of a stand-alone homology retrieval apparatus. As shown in FIG. 10, the homology retrieval system 1 includes a data input/output unit 12, a homology retrieval unit 13, and a storage device 37. The data input/output unit 12 includes a computer device including a CPU 31 that executes a program capable of executing various steps with a computer, an input/output I/F (interface) 32, an input device 33 that performs data input and an output device 34 that performs data output. The homology retrieval unit 13 includes a computer device including a program storage unit 36 in which a program is stored, and a CPU 35 that executes the program. In the storage device 37, the sequence information before compression of a query sequence and a target sequence, the sequence information after compression thereof, information on the number of consecutive identical bases, the degree of similarity, and data on the order of candidate sequences are stored, for example. It should be noted that the data input/output unit 12, the homology retrieval unit 13 and the storage device 37 merely represent functions, and they may be integrated into a single computer device, or may be separately configured as a plurality of computer devices, for example.

### Configuration Example of Second System

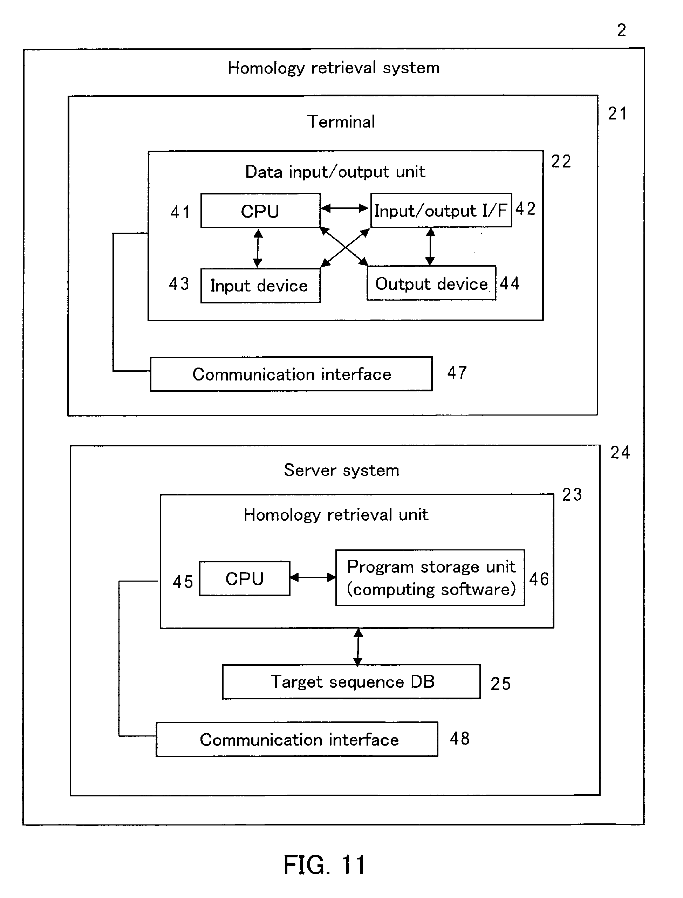

FIG. 9 shows an overall configuration of a network-type system that executes processing with a server. As shown in FIG. 9, a homology retrieval system 2 according to this embodiment includes a terminal 21 and a server system 24. The terminal 21 includes a data input/output unit 22. The server system 24 includes a homology retrieval unit 23 and a database (target sequence DB) 25 in which target sequences are stored. The homology retrieval unit 23 includes, for example, a compressed sequence preparation unit (e.g., a compressing conversion unit), a compressed candidate sequence retrieval unit, a consecutive identical base number preparation unit (e.g., a consecutive base number counting unit), a similarity degree computing unit, and a candidate sequence selection unit. The homology retrieval unit 23 and the server system 24 are connected, for example, via a communication line 100 such as a public network that functions as the internet based on TCP (Transmission Control Protocol)/IP (Internet Protocol) or a private line. FIG. 11 shows an example of the configuration of an apparatus of the above-described network-type system. The terminal 21 includes a data input/output unit 22 and a communication interface 47, and is connected to a communication line via a communication interface 47. The data input/output unit 22 includes a CPU 41 that executes a program, an input/output I/F 42, an input device 43 that performs data input and an output device 44 that performs data output. The data input/output unit 22 and communication interface 47 described above merely represent functions, and they may be integrated into a single computer device, or may be separately configured as a plurality of computer devices, for example. The server system 24 includes a homology retrieval unit 23, a database (target sequence DB) 25 in which target sequences are stored, and a communication interface 48, and is connected to the communication line via a communication interface 48. The homology retrieval unit 23 includes a CPU 45 that executes a program capable of executing a series of steps for selecting a candidate sequence, and a program storage unit 46 in which the program is stored. The homology retrieval unit 23, the target sequence DB 25 and the communication interface 48 merely represent functions, and they may be integrated into a single computer device, or may be separately configured as a plurality of computer devices, for example.

### Embodiment 3

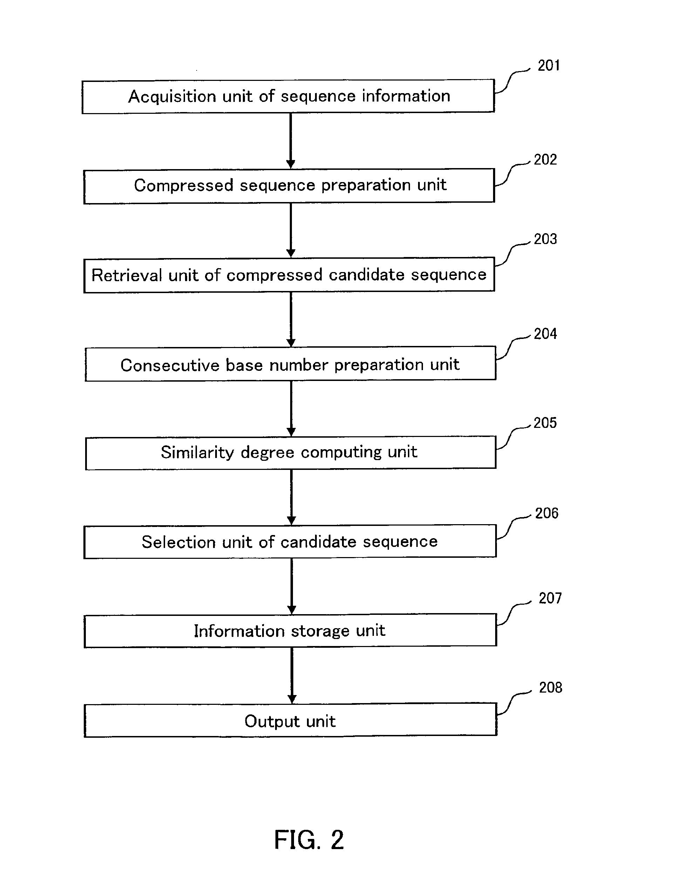

In the following, an example of a homology retrieval system according to the present invention will be described. FIG. 2 is a diagram schematically showing the configuration of a homology retrieval system according to this embodiment. It should be noted that the present invention is not limited to this embodiment, and various modifications can be made without departing from the gist of the invention.

As shown in FIG. 2, the homology retrieval system according to this embodiment includes a sequence information acquisition unit (input unit) 201, a compressed sequence preparation unit 202, a compressed candidate sequence retrieval unit 203, a consecutive identical base number preparation unit 204, a similarity degree computing unit 205, a candidate sequence selection unit 206, an information storage unit 207, and an output unit 208. One example of this homology retrieval system is a homology retrieval apparatus configured with a computer system having the above-described hardware configuration. Each of the constituent unit may be, for example, a functional block that is realized by a CPU of a computer executing a predetermined program. Therefore, each of the constituent unit may not be implemented as hardware, and may be the above-described network system.

The sequence information acquisition unit 201 has a function of acquiring the sequence information of the nucleic-acid base sequence of a target sequence and the nucleic-acid base sequence of a query sequence. This information acquisition can be performed, for example, through input performed by the above-described input device. Alternatively, it is possible to access an external network such as the internet, and to acquire information from an external database or the like, as described above. When the above-described information is obtained from an external network, it is possible, for example, to download database information onto a storage device (e.g., the RAM 102, the information storage unit 113 or the like in FIG. 1) of a computer, or to use the above-described information in a state in which the communication line remains connected. There is no limitation on the external database from which the information is obtained. It is also possible to use a removable recording medium in which the sequence information is stored.

For example, the compressed sequence preparation unit 202 has a function of converting the sequence information of a nucleic-acid base sequence into a compressed sequence in which a homopolymer region including two or more repeating identical bases is replaced by a single base of the bases (compressing conversion unit). That is, the compressed sequence preparation unit 202 generates the sequence information of a compressed sequence in which a homopolymer region including two or more repeating identical bases is replaced by a single base of the bases for a target sequence and a query sequence that have been acquired by the sequence information acquisition unit 201.

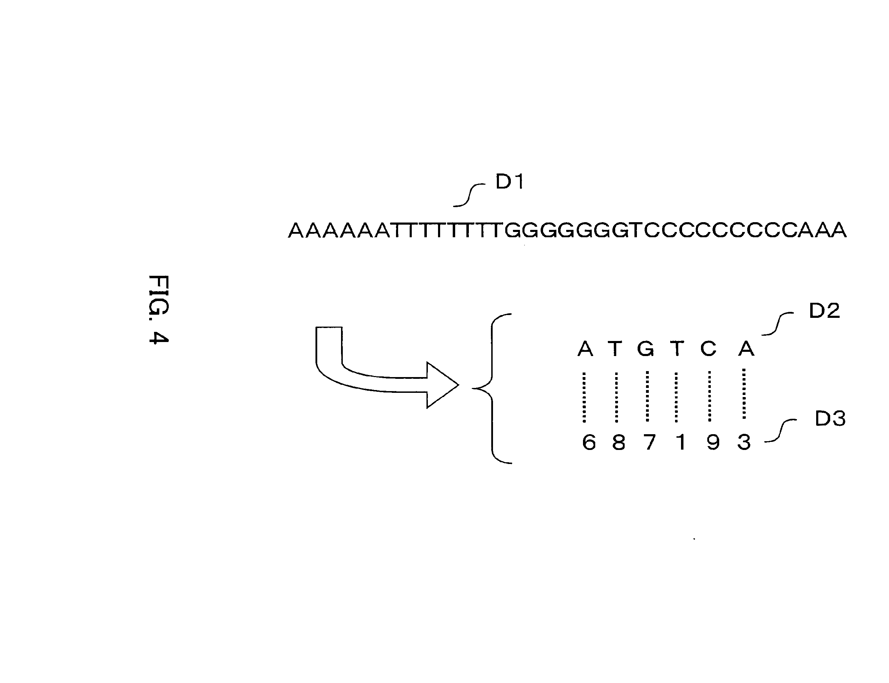

Here, an example of the compressing conversion of a nucleic-acid base sequence is described with reference to FIG. 4. FIG. 4 schematically shows compressing conversion and counting of the number of consecutive identical bases, which will be described later. In FIG. 4, D1 is a nucleic-acid base sequence. This nucleic-acid base sequence has regions in which identical bases are lined up successively. Specifically, the nucleic-acid base sequence has, starting from the left end (the 5' end), a region including 6 consecutive adenines, a region including 8 consecutive thymines, a region including 7 consecutive guanines, a single thymine in between, a region including 9 consecutive cytosines, and a region including 3 consecutive adenines. Each of these regions including consecutive identical bases is a "homopolymer region" in the present invention. In compressing conversion, a region including a plurality of (two or more) consecutive (repeating) identical bases in this way is regarded as a single base, and a sequence showing only an arrangement of 4 types of bases is generated. In FIG. 4, the sequence indicated by D2 corresponds to a compressed sequence for the nucleic-acid base sequence indicated by D1.

As described above, in a commonly used homology retrieval, a genomic-scale target sequence is generally broken down into partial sequences before being retrieved, so that it is also preferable to divide a target sequence into partial sequences before it is subjected to a retrieval in the present invention. Therefore, a compressed sequence of a target sequence according to the present invention may be a full-length compressed sequence, but is preferably a compressed target partial sequence group as described above. There is no limitation on the generation of partial sequences from a target sequence, and any conventionally known method may be used. Specific examples thereof include generating a compressed target partial sequence group by sequentially shifting one base at a time from the top of a target sequence after compression. That is, with the system of this embodiment, when the sequence information of a target sequence is acquired by the acquisition unit 201, the sequence information of a compressed target sequence can be generated in the compressed sequence preparation unit 202, and the sequence information of a compressed target partial sequence group made up of a compressed target partial sequence resulting from further dividing the compressed target sequence into fixed lengths can be generated. In the case of retrieving a complementary strand of the target sequence as well, for example, the sequence information of the compressed target partial sequence group and the information on the compressed target partial sequence group of its complementary strand may be acquired in an alternating manner. The sequence information of the compressed target partial sequence group of the latter can be readily determined by reversing the order of the arrangement of the bases in the compressed target partial sequence of the former for the complementary bases. Further, information on the number of consecutive bases, which will be described later, can be acquired, for example, by reversing the order of a string sequentially showing the number of consecutive identical bases in each of the homopolymer regions of the former for the complementary bases. For example, if the arrangement of the numbers of consecutive bases of the former is "6-8-7-1-9-3", then the arrangement of the numbers of consecutive bases of the latter will be "3-9-1-7-8-6".

The compressed candidate sequence retrieval unit 203 has the following function. That is, first, a compressed sequence of a target sequence (compressed target sequence) and a compressed sequence of a query sequence (compressed query sequence) that have been generated in the compressed sequence preparation unit 202 are compared. Then, a resolution retrieval is performed for the compressed target partial sequence in the compressed target sequences that matches the compressed query sequence, and the resolved compressed target partial sequence is selected as a compressed sequence of a candidate sequence (compressed candidate sequence).

For example, the identical base consecutive base number preparation unit 204 has a function of counting the number of consecutive identical bases in a homopolymer region for the compressed candidate sequence and the compressed query sequence that have been selected by the compressed candidate sequence retrieval unit 203 (consecutive base number counting unit). It should be noted that information on the number of consecutive bases that has been counted outside the system may be input as described above.

Here, an example of counting the number of consecutive identical bases will be described with reference to FIG. 4 described above. As described above, in FIG. 4, D1 is a nucleic-acid base sequence before compression, and D2 is a compressed sequence resulting from compressingly converting the nucleic-acid base sequence. The number of consecutive occurrence of each base in the compressed sequence D2 corresponds to the above-described consecutive base number according to the present invention. It should be noted that in this embodiment, the number of bases in the homopolymer regions is counted as the consecutive base number (the first base is also counted), and a non-repeating base is counted as a single base. The information on the number of consecutive identical bases can be represented, for example, as the number of consecutive identical bases in the string D3 "687193" as shown in FIG. 4. The number of elements in the compressed sequence D2 "ATGTCA" and the number of elements in the number of consecutive identical bases in the string D3 "687193" are the same, and the number of elements in the nucleic-acid base sequence D1 before compression is reduced to the number of elements in the compressed sequence D2 by the number of homopolymer regions that appear.

The similarity degree computing unit 205 has a function of comparing the above-described number of consecutive bases between the compressed query sequence and the compressed candidate sequence for each corresponding base, based on the information on the number of consecutive bases counted by the identical base consecutive base number preparation unit 204, and computing the degree of similarity indicating the homology of the candidate sequence with the query sequence from the degree of match or the degree of mismatch in the number of consecutive bases.

The candidate sequence selection unit 206 has a function of determining the homology ranking of the candidate sequence with the query sequence by comparing the results for the degree of similarity computed by the similarity degree computing unit 205, and selecting an arbitrary number of candidate sequences having a relatively high homology.

The information storage unit 207 has a function of storing an arbitrary number of candidate sequences having relatively high homology with the query sequence selected by the candidate sequence selection unit 206 and the degree of similarity to the query sequence. Particularly, when a plurality of candidate sequences are retrieved by the compressed candidate sequence retrieval unit 203, it is preferable, for example, to store an arbitrary number of degrees of similarity of candidate sequences having high homology, and, when a new degree of similarity has been computed, to compare the stored plurality of degrees of similarity and the new degree of similarity, and to again determine the ranking, select an arbitrary number of candidate sequences having high homology, and store the information thereof. This makes it possible to further retrieve a partial sequence of the target sequence that has higher homology with the query sequence.

The output unit 208 has a function of outputting the information stored in the information storage unit 207. For example, when the homology retrieval system includes a display unit (display device) such as a display, the information may be displayed on the display screen, or may be displayed to the outside by printing it out with a printer. Alternatively, the information may be output, for example, to a storage device (e.g., an information storage unit) of a computer, or a removable recording medium, and stored therein.

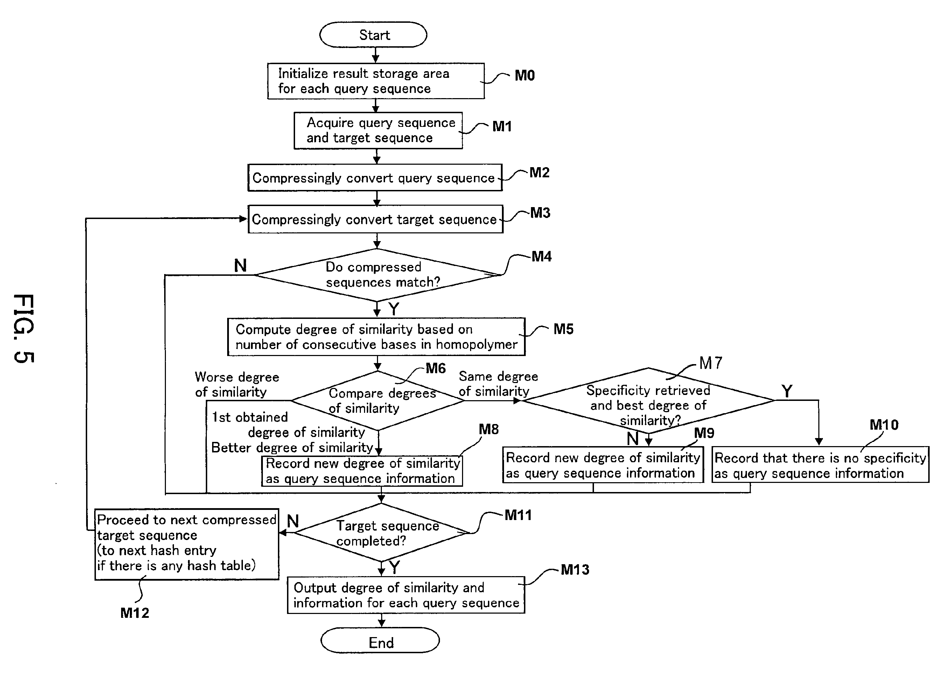

Next, an example of a processing flow in the homology retrieval system according to this embodiment will be described with reference to FIG. 5. FIG. 5 is a flowchart illustrating the processing flow. This processing is an example of a homology retrieval method according to the present invention, and can be executed, for example, by a homology retrieval system of the invention and a computer program of the invention. It should be noted that a compressed sequence of a target sequence (compressed target sequence) is described as the compressed target partial sequence group described above.

First, the processing starts with the initialization of a result storage area in which the result for each query sequence is stored (step M0). Subsequently, the sequence information of a query sequence and a target sequence is acquired (input) (step M1). For example, by directly inputting the sequence information through the input device 107 such as a mouse, or indirectly specifying a file or a location on a network where the target sequence is stored, the following processing is started for sequence information that has been taken into the system (e.g., the RAM 102 or the storage device 103).

Then, the acquired sequence information of the query sequence is compressingly converted into a compressed sequence (step M2), and the acquired sequence information of the target sequence is compressingly converted into a compressed sequence (compressed target partial sequence group) (step M3). The compressing conversion of the query sequence and the target sequence (steps M2 and M3) may be performed separately in a random order, or may be performed in parallel. When there is a plurality of query sequences, the compressing conversion for all the query sequences may be completed before executing the step described below, or step M2 and the step described below may be performed in parallel by sequentially subjecting the compressingly converted sequences to the step described below. Similarly, for the target sequence, the compressing conversion of all the target partial sequences included in the target partial sequence group may be completed before performing the step described below, or step M3 and the step described below may be performed in parallel by sequentially subjecting the compressingly converted sequences to the step described below. Here, when there is a plurality (especially, a large number) of query sequences, it is preferable to generate a hash table. Using a hash table allows, for example, even approximately 1,000,000 to 5,000,000 query sequences to be processed speedily. In addition, it is also preferable to generate a hash table for a compressed target partial sequence group for a target sequence since it is of a genomic-scale. The generation of these hash tables will be described later.

Subsequently, the compressed sequence of the query sequence (compressed query sequence) and the compressed target partial sequence group of the target sequence are compared, and the compressed target partial sequence that matches the compressed query sequence is retrieved (step M4). Then, if a compressed target partial sequence that matches the compressed query sequence can be retrieved, then that compressed target partial sequence is selected as the compressed target partial sequence of a candidate sequence (hereinafter, also referred to as a "compressed candidate sequence"). On the other hand, if a compressed target partial sequence that matches the compressed query sequence cannot be retrieved, then the procedure moves to step M11.

According to the present invention, compressed sequences for which the number of consecutive identical bases in a homopolymer region, which has been the problem, are not taken into consideration are first compared in this way, and the matching candidate sequence is selected. Therefore, it is possible to avoid conventional problems, including, for example, that of determining that there is no similarity simply due to a difference in the number of consecutive bases, and conversely, that of determining that there is similarity even in the case where there is a mismatch other than that in the number of consecutive identical bases in a homopolymer region, because of excessive consideration of the problem of the number of consecutive bases. Since there is a match in at least the arrangement of the base types, a homology retrieval can be performed accurately simply by determining homology with regard to the number of consecutive bases as described below, and determining the ranking (order) in homology of the candidate sequences.

Then, the degree of similarity indicating the homology of the compressed candidate sequence selected at step M4 with the compressed query sequence is computed (step M5). To compute the degree of similarity, information on the number of consecutive identical bases in homopolymer regions is required for each of the compressed candidate sequence and the compressed query sequence. Therefore, in order to compute the degree of similarity, first, the number of consecutive identical bases in the homopolymer regions is counted for the compressed candidate sequence and the compressed query sequence at step M5. Specifically, from the nucleic-acid base sequence of the compressed candidate sequence before compression and the nucleic-acid base sequence of the compressed query sequence before compression, the number of consecutive identical bases in the homopolymer regions is counted. The counting of the number of consecutive bases in the query sequence may be performed at this time, or may be performed, for example, in sequence or in parallel during compressing conversion. The counting of the number of consecutive bases in partial sequences of the target sequence may be performed, for example, in sequence or in parallel during compressing conversion as in the case of the query sequence, or the counting may be performed only for the compressed candidate sequence selected at step M4 instead of performing the counting for all the target partial sequences, because it is necessary to obtain a result for the candidate sequence.

The number of consecutive identical bases in homopolymer regions may be counted in advance, and the information thereof may be, for example, directly input, or read from a storage device, an external recording medium or the like as necessary. The method for computing the degree of similarity will be described later.

Then, the result for the degree of similarity obtained at step M5 is compared with another degree of similarity (step M6). Ordinarily, when a plurality of target partial sequences that exhibit high homology with the query sequence are obtained in a homology retrieval, determination of ranking in homology, or selection of a target partial sequence having high homology (i.e., a target partial sequence having high specificity) is performed. Accordingly, when a compressed candidate sequence that matches the compressed query sequence is retried at step M4, for example, the homology ranking is determined by comparing the homology of candidate sequences with the query sequence, and an arbitrary number of candidate sequences having high homology are also selected in the present invention. As described above, the number of the candidate sequences selected is not limited, and can be set to an arbitrary number.

The subsequent step of step M6 can be processed according to the comparison result obtained at step M6 in the following manner.
(1) If the result obtained at step M6 (the current degree of similarity) is a first obtained degree of similarity, the procedure moves to step M8, and the current degree of similarity is recorded as a result for the query sequence in the initialized result storage area for query sequences (step M8). Examples of the information recorded include, in addition to the degree of similarity of a candidate sequence with the query sequence, the type of target sequence (e.g., the genome type and the chromosome type), the types of the strand (forward strand or the reverse) of the target sequence, and the coordinates of the candidate sequence in the target sequence (the same applies to the following).
(2) If the result for the current degree of similarity obtained at step M6 is the second or a further result, and the number of candidate sequences has not reached the arbitrary number, this result is further recorded in the result storage area for query sequences (step M8). At this time, the candidate sequences are ranked with respect to the query sequence based on the degree of similarity.
(3) If the result obtained at step M6 (the current degree of similarity) is the second or a further result, and the number of candidate sequences has reached the arbitrary number, the candidate sequences are again ranked with respect to the query sequence based on the current degree of similarity and the similarities that have been already recorded, then an arbitrary number of the candidate sequences from the top of the ranking are selected, and the information thereof is recorded as a substitute (step M8). Those candidate sequences that were not selected are subjected to step M11.
(4) If the result obtained at step M6 (the current degree of similarity) does not indicate homology with the query sequence or indicates extremely low homology, the procedure moves to step M11.

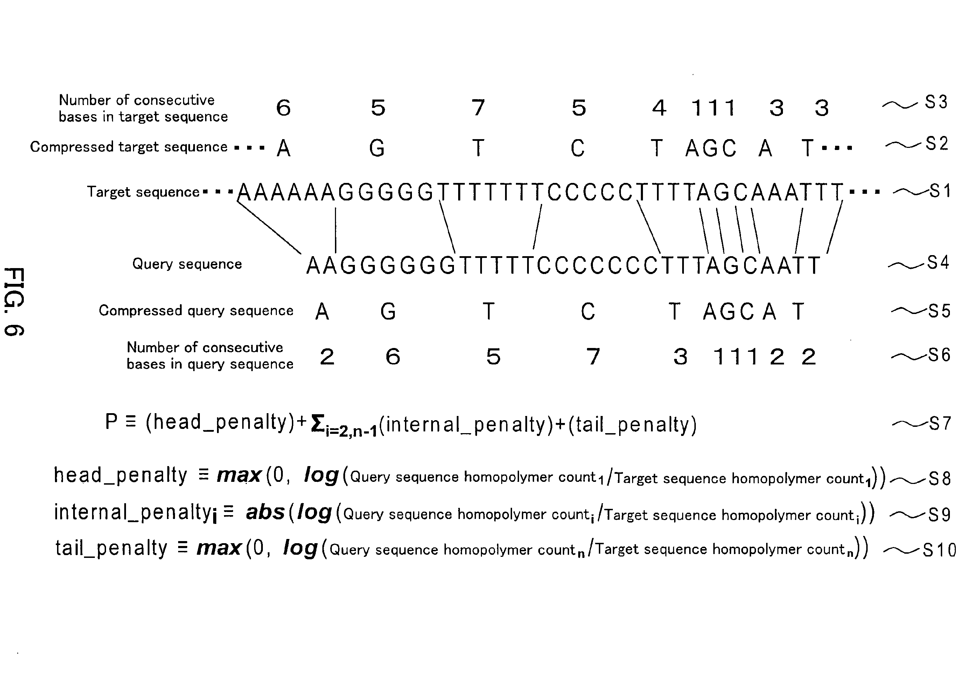

When a retrieval is performed for determining whether a candidate sequence is homologous specifically only with a certain query sequence, the subsequent step of step M6 can be processed according to the comparison result at step M6 in the following manner.
(5) If the current degree of similarity obtained at step M6 is the second or a further result, and is a degree of similarity indicating higher homology than the recorded similarity (especially if the query sequence and the candidate sequence completely match), the current degree of similarity is recorded as the best degree of similarity (step M8).
(6) If the current degree of similarity obtained at step M6 is the second or a further result, and is the same degree of similarity as the recorded degree of similarity, determination as to whether there is specificity with the query sequence (specificity retrieval) is performed (step M7). That is, if the current degree of similarity and the recorded degree of similarity are the same, whether this degree of similarity is the best degree of similarity (the best value) is determined. For example, in accordance with a similarity degree calculation method using a penalty score, which will be described later with reference to FIG. 6, the best value may be "0.0 (minimum value)", and this means that the query sequence and the candidate sequence completely match. The presence of two or more candidate sequences that completely match the query sequence means that the query sequence consequently does not exhibit specificity for the target sequence. Accordingly, in such a case, information indicating that the query sequence does not have specificity for the target sequence is recorded (step M10). It is clear that the query sequence does not have specificity for these candidate sequences, so that these candidate sequences do not need to be included in further retrievals. Accordingly, in the case of retrieving a plurality of query sequences, the data of such an above-mentioned query sequence may be deleted. Furthermore, it is also clear that these candidate sequences do not have specificity for the above-mentioned query sequence, so that these candidate sequences do not need to be included in further retrievals. Accordingly, after recording that these candidate sequences do not have specificity for the target sequence, the candidate sequences may be deleted from the data that is to be retrieved. By reducing the data to be retrieved in this way, the system performance, for example, is further improved in reverse proportion to the data reduced. On the other hand, if the current degree of similarity and the recorded degree of similarity are the same, but the current degree of similarity does not have the best value (best score) as described above, the current score is recorded in the result storage area for query sequences (step M9).

Then, if it is determined at step M11 that the retrieval of the prepared target sequence has been completed for a particular query sequence, the recorded degrees of similarity and other information for that query sequence are output (step M13), and if it is determined that the retrieval in correlation with the target sequence has been completed, the retrieval is terminated. On the other hand, if the retrieval in association with the prepared target sequences for a certain query sequence is not completed at step M11, the procedure proceeds to compressing conversion of the subsequent target sequence (step M3) or comparison with the compressed target partial sequence group that has been compressingly converted (step M4)(step M12).

If the retrieval of the prepared target sequence has been completed for a particular query sequence, and another query sequence is prepared, the same processing is performed for that query sequence. Here, when there is a plurality of query sequences, the processing series of step M0 to M13 may be completed for a particular query sequence before processing another query sequence, or the processing series may be performed in sequence or in parallel. Then, after completion of the retrieval, the stored information (e.g., the degree of similarity, and the coordinates in the target sequence) may be output for each query sequence.

### Embodiment 4

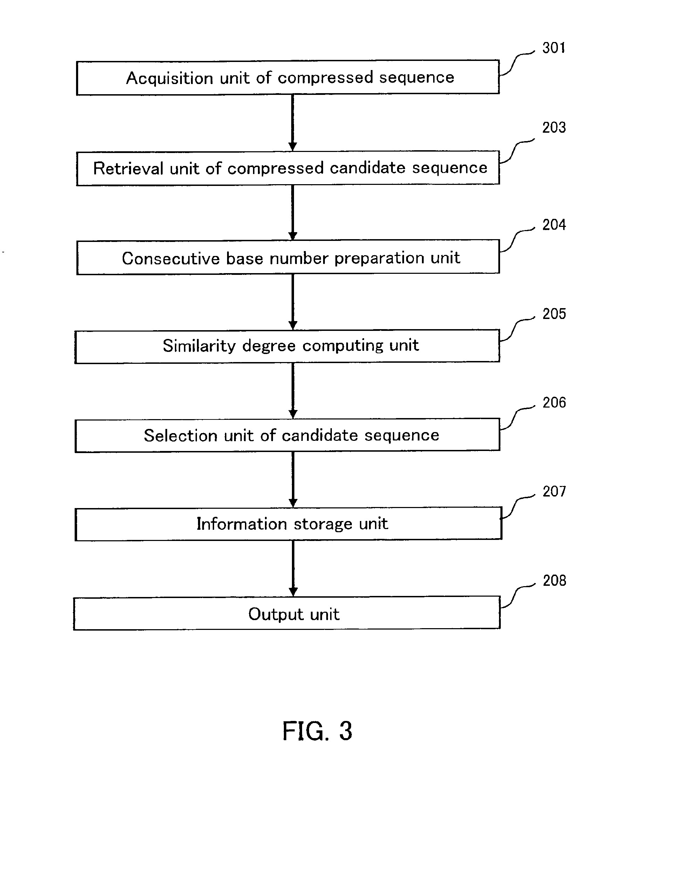

In the following, another example of the homology retrieval system according to the present invention will be described. FIG. 3 is a block diagram schematically showing the configuration of a homology retrieval system according to this embodiment. It should be noted that this system is the same as the system of Embodiment 3 shown in FIG. 2, unless otherwise indicated.

This homology retrieval system includes a compressed sequence acquisition unit 301 in place of the sequence information acquisition unit 201 and the compressing conversion unit 202 of the system of Embodiment 3. Thus, in the homology retrieval system of this embodiment, the sequence information of a compressed query sequence and a compressed target sequence that have been compressingly converted in advance may be acquired (input).

### Embodiment 5

An example of the similarity degree calculation according to the present invention will be described with reference to FIG. 6. It should be noted that the present invention is not limited to the following details, and various modifications may be made without departing from the gist of the present invention.

FIG. 6 shows an example of a list of information necessary for calculating a degree of similarity. In FIG. 6, S1 to S3 are information relating to a target sequence, and S4 to S6 are information relating to a query sequence. S1 is the target sequence before compression, S2 is the compressed target sequence, and S3 is a string indicating the number of consecutive bases in the target sequence. Similarly, S4 is the query sequence before compression, S5 is the compressed query sequence, S6 is a string indicating the number of consecutive bases in the query sequence.

In a homology retrieval system according to the present invention, first, the compressed target partial sequence (S2) of the target sequence that matches the compressed query sequence (S5) is retrieved by comparing a compressed sequence of the target sequence (compressed target sequence) and a compressed sequence of the query sequence (compressed query sequence) as described above. As shown in FIG. 6, the compressed query sequence (S5) and the compressed target sequence (compressed target partial sequence S2) show the same arrangement of the four base types.

In this embodiment, an example is described in which the degree of similarity is computed by using the degree of mismatch in the number of consecutive bases for corresponding bases between the compressed query sequence and the compressed target partial sequences as a penalty score, and adding the penalty score of each corresponding base. Here, as will be described later, a mismatch where the number of consecutive bases in the upstream terminal base or the downstream terminal base of the compressed query sequence before compression is less than the number of consecutive bases in the upstream terminal base or the downstream terminal base of the compressed candidate sequence is excluded. In the case of using a degree of mismatch as an index of homology in this way, it can be determined that a relatively large value indicates relative non-similarity and a relatively small value indicates relative similarity, for example.

To compute the penalty score for each corresponding base, the penalty score of the upstream terminal base, the penalty score of the downstream terminal base, and the penalty score of internal bases other than the two terminal bases are separately calculated in the compressed target sequence (S2) and the compressed query sequence (S5). Then, the sum total of these (the sum total penalty) is used as the degree of similarity indicating homology. In the following, computation of each of the penalty scores will be described.

Expressions S8 to S10 shown in FIG. 6 are an expression (S8) for computing the penalty score of the upstream terminal end, an expression (S9) for computing the penalty scores of the internal bases, and an expression (S10) for computing the penalty score of the downstream terminal end, respectively. Also, expression S7 is an expression for computing the sum total penalty, and an index indicating the homology (degree of similarity) between the query sequence (S4) and the partial sequence of the target sequence (S1) is obtained thereby. Additionally, in expressions S8 to S10 of FIG. 6, the homopolymer count means the number of consecutive identical bases in a single homopolymer region. In expression S8, the homopolymer count₁ is the number of consecutive bases in the first corresponding base type (the upstream terminal base type) in the compressed target sequence (S2) and the compressed query sequence (S5). In expression S9, the homopolymer countᵢ is the number of consecutive bases in the ith corresponding (i is n-1, and n is an integer of 3 or more) base type in the two compressed sequences (S2 and S5). In expression S10, the homopolymer countₙ is the number of consecutive bases in the last corresponding (nth, n is an integer of 3 or more) base type (the downstream terminal base type) in the compressed target sequence (S2) and the compressed query sequence (S5).

First, expression S9 for determining the penalty scores for the internal bases will be described. In the case of this expression, when the number of consecutive bases in a query sequence and the number of consecutive bases in a target sequence are the same for a certain base (query sequence homopolymer count = target sequence homopolymer count), the value obtained by dividing the former by the latter is 1, and therefore, the natural logarithm thereof is 0. Further, when the number of consecutive bases in a query sequence is more than the number of consecutive bases in a target sequence (query sequence homopolymer count > target sequence homopolymer count), the value obtained by dividing the former by the latter is more than 1, and therefore, the natural logarithm thereof takes a positive value. On the other hand, when the number of consecutive bases in a query sequence is less than the number of consecutive bases in a target sequence for a certain base (query sequence homopolymer count < target sequence homopolymer count), the value obtained by dividing the former by the latter is 1 or less and, therefore, the natural logarithm thereof takes a negative value. That is, the closer the number of consecutive bases in the query sequence and the number of consecutive bases in the target sequence become, the more the natural logarithm approaches 0, whereas the more different the number of consecutive bases in the query sequence and the number of consecutive bases in the target sequence become, the greater the value of the natural logarithm deviates from 0. Accordingly, in expression S9, the absolute value of the natural logarithm is taken as a penalty score.

Next, expression S8 and expression S10 will be described. Also for the terminal bases, when the number of consecutive bases in a query sequence and the number of consecutive bases in a target sequence are the same (query sequence homopolymer count = target sequence homopolymer count), the value obtained by dividing the former by the latter is 1 and, therefore, the natural logarithm thereof is 0 as in the case of expression S9 described above. Further, when the number of consecutive bases in a query sequence is more than the number of consecutive bases in a target sequence (query sequence homopolymer count > target sequence homopolymer count), the value obtained by dividing the former by the latter is more than 1 and, therefore, the natural logarithm thereof takes a positive value. However, when the number of consecutive bases in a query sequence is more than the number of consecutive bases in a target sequence, i.e., the number of consecutive bases in the target sequence is less, the following concept is applied to the terminal bases. Since the query sequence is a partial sequence in a genome or a chromosome, for the terminal homopolymer regions, it is not appropriate to evaluate the homology as poor simply because the number of consecutive bases in a query sequence is less than the number of consecutive bases in a target sequence. Therefore, for the number of consecutive bases in the terminal bases, expressions (S8 and S10) are used in which the penalty score is taken as 0 in the case where the number of consecutive bases in a target sequence is less than the number of consecutive bases in a query sequence, and the case where these number of consecutive bases are the same, and a computation is performed only in the case where the number of consecutive bases in a query sequence is larger, that is, the natural logarithm takes a positive value, instead of taking the absolute value as in expression S9.

Then, in expression S7, the penalty score of the upstream terminal end that has been calculated in expression S8, the sum of the penalty scores of the internal bases that has been calculated in expression S9, and the penalty score of the downstream terminal end that has been calculated in expression S10 are added to obtain an index indicating the homology (degree of similarity) between the query sequence (S4) and the partial sequence (S1) of the target sequence. It should be noted that in this example, "0" represents the maximum degree of match, i.e., a perfect match, and the more the value increases relatively, the more the degree of similarity decreases relatively. Ordinarily, the upper limit threshold is set to, for example, the natural logarithm of 2, and a value exceeding this may be regarded as indicating no homology.

Expressions S7 to S10 represent an example realizing the present invention, and the invention is not limited thereto. The reason that logarithms are used as an example as described above is to additively represent the accumulation of error penalties, and these expressions can be modified into equivalent expressions or applied expressions, for example, by reversing the numerator and the denominator, reversing the positive and negative values, either taking the natural logarithm or handling as an exponent, either using the natural logarithm or using another value as the base of a logarithm, or either taking the absolute value or the square root in each of the expressions. Furthermore, depending on the applications of the present invention, modifications such as weighting according to the location are also possible. Those skilled in the art would be able to perform such modifications and configurations of the expression based on the descriptions in the present specification.

As a specific example, the evaluation of overcall and undercall can be incorporated by weighting according to the difference between positive and negative values before taking the absolute value in expression S7. As for errors in the number of identical bases in a homopolymer region, for example, the possibility that the base number tends to be counted higher or lower depending on the base sequencing method has been suggested. Therefore, by taking this tendency into consideration, the influence on the penalty score due to such errors can be further reduced. For example, when a tendency for the number of identical bases on the query sequence side to be greater is known in advance, the following processing is possible. That is, when calculating the penalty score for each base using, for example, the expressions shown in FIG. 6, if the value before taking the absolute value is positive, i.e., the number of consecutive bases on the query sequence side is larger than the number of consecutive bases on the target sequence side (query sequence homopolymer count > target sequence homopolymer count), and this is multiplied by a coefficient less than 1, for example. This can reduce the apparent penalty score which could be increased due to a tendency for the number of identical bases of the query sequence to be larger. That is, by reflecting an "overcall tendency", it is possible to obtain a more highly reliable degree of similarity. On the other hand, for example, when a tendency for the number of identical bases on the query sequence side to be less is known in advance, the following processing is possible. That is, when calculating the penalty score for each base using, for example, the expressions shown in FIG. 6, the value before taking the absolute value is negative, i.e., the number of consecutive bases on the query sequence side is less than the number of consecutive bases on the target sequence side (query sequence homopolymer count < target sequence homopolymer count), and this is multiplied by a coefficient less than 1, for example. This can reduce the apparent penalty score that could be increased due to a tendency for the number of identical bases in the query sequence to be less. That is, by reflecting "undercall tendency", it is possible to obtain a more highly reliable degree of similarity. It should be noted that addition is performed only for positive values in both of expression S8 (head_penalty) and expression S10 (tail_penalty), as described above, and it is always the case that a coefficient of less than 1 is simply multiplied in the former case (where the value before taking the absolute value is positive).

Furthermore, a tendency in the number of identical bases relating to the base types (A, G, C, T) also can be weighted in the same manner as described above. That is, when calculating the penalty score of each base using expression S9, which is a partial expression of expression S7, it is also possible to reflect, for example, the evaluation of overcall and undercall tendencies for each base type by changing the weighting factor relating to the positive and negative values before taking the absolute value for each base type.

### Embodiment 6

An example of a target sequence hash table according to the present invention will be described. It should be noted that the present invention is not limited to the following details, and various modifications can be made without departing from the gist of the invention.

A hash table is a data structure in which the elements in the table are directly indexed by values resulting from multiplying the character string of the compressed target partial sequences of a compressed target partial sequence group by a hash function, for example. The method for obtaining values resulting from multiplying the character strings by a hash function is not limited, and a conventionally known approach can be used. As a specific example, this can be readily realized by using the approach defined in the hash Code method of the standard package class of java. lang. String, which is included in the programming language, java. Furthermore, it is possible to acquire the necessary indexes by further dividing a value mapped into an integer space by the number of table elements to calculate a positive remainder.

Even different character strings may be assigned to the same hash table index through a value collision. In this case, for example, it is preferable to secure an overflow area corresponding to the length of a target sequence after compression. Then, it is possible to successively access the elements in the target sequence for a particular hash index in a direct manner, for example, by adopting a data structure that sequentially indicates the collided character string elements. Ordinarily, blank data indicating completion is stored in the last element in a hash table. Ordinarily, this is preferably placed in a RAM serving as the main storage device. However, if the capacity exceeds physical limits, it may be placed, for example, in an external storage device, and be cached into the main storage device as needed.

By generating a target sequence hash table that includes an overflow area in advance in this way, it is possible to combine, for example, the acquisition of a compressed target sequence (step M3 of FIG. 5) with the retrieval of the compressed target partial sequence that matches the compressed query sequence (step M4 of FIG. 5). Although the target sequence is of genomic-scale and thus is an extremely long sequence, by using a hash retrieval using a hash table, it is possible to skip a compressed target partial sequence that mismatches the compressed query sequence, and acquire the next compressed target partial sequence by a single logical access. Furthermore, by generating a hash table, it is possible to acquire the next target sequence (compressed target sequence group) at a higher speed by proceeding to the next hash entry in the target sequence hash table, for example, when the retrieval of a certain target sequence is completed in step M11 as shown in FIG. 5.

The above-described hash table is preferably a data structure in which the homopolymer count can be correlated as necessary, for example, with the sequence of a target partial sequence before compression, the coordinates in a target sequence before compression, the number of consecutive identical bases in each homopolymer region of the target partial sequence, and the like. In view of system life cycle, such a hash table is not particularly necessary, for example, for a target sequence that requires only a single retrieval, and it is sufficient, for example, to perform only compression processing as necessary, and to correlate the homopolymer count with a compressed query sequence. Even if a target sequence hash table is not generated, this will not hinder the performance improvement achieved by a query sequence hash table, which will be described later.

There is no limitation on the hash table, and a reference can be made to Donald Knuth. "The Art of Computer Programming" Volume 3, Sorting and Searching, second edition, 1998, pp. 513-558, ISBN 0-201-89685-0, for example. Besides a hash retrieval, a binary tree retrieval and a B-tree retrieval can be used in the present invention as described above, and a reference can be made to, respectively, Donald Knuth. Fundamental Algorithms, Third Edition. Addison-Wesley, 1997. pp. 318-348. ISBN 0-201-89683-4. and R. Bayer and E. McCreight. "Organization and Maintenance of Large Ordered Indexes," Acta Informatica, 1, 1972, for example. It should be noted that a hash retrieval, a binary tree retrieval, and a B-tree retrieval are given as examples, and the present invention is by no means limited thereto.

### Embodiment 7

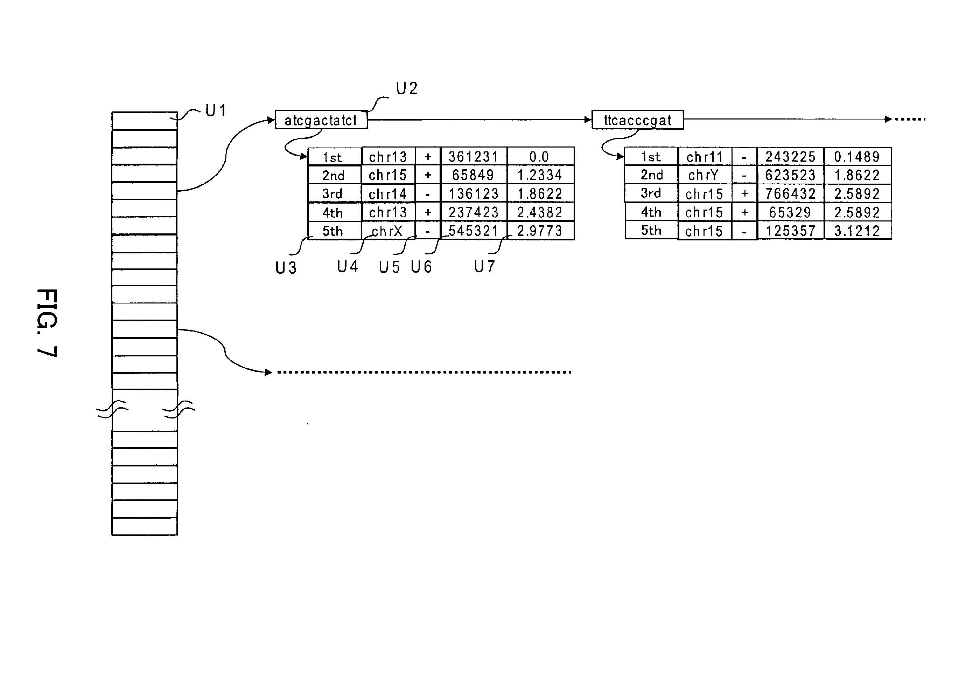

An example of a query sequence hash table according to the present invention will be described with reference to FIG. 7. It should be noted that the present invention is not limited to the following details, and the hash table can be generated in the same manner as in Embodiment 6, unless otherwise indicated.

FIG. 7 shows an example of the structure of a query sequence hash table. In this hash table, the elements in the table are directly indexed by values resulting from multiplying the character string of each query sequence by a hash function, for example. As described above, with regard to collisions, it is preferable to form an overflow area, for example, by chaining with the next element.

As described above, a retrieval result for the query sequence is preferably recorded with the progress of retrieval. It is therefore preferable to generate, for example, a table in which various retrieval information is stored along with a query sequence hash table. The above-described information storage table includes, for example, the homology ranking based on the degree of similarity (U3), the chromosome number of the target sequence that is homologous with the query sequence (U4), the type of strand of the target sequence (U5), the position on the chromosome of the target sequence (target partial sequence) before compression (U6), and the degree of similarity (U7).

Further, as shown in step M7 (FIG. 5) described above, in the case of performing a specificity retrieval, it is preferable to delete data in the query sequence hash table as needed. As described above, a specificity retrieval is, for example, for examining whether there is only one target partial sequence that shows a score of the highest homology with the query sequence in the target sequence. Accordingly, upon retrieval of a plurality of scores of the highest homology (the top scores) from among the partial sequence group of the target sequence, it is clear that there is no specificity. Therefore, when a score of the highest homology is obtained for a plurality of target partial sequences for the target sequence like this, it is preferable to exclude those elements (query sequences) from the hash table. This makes it possible, for example, to avoid a memory shortage, thereby reducing search time in an overflow area. In the example of the scoring system shown in FIG. 7, this corresponds to the case where a plurality of the top elements with the same score showing a degree of similarity (U7) of, for example, 0.0, are present. Excluding elements indicating such a result in the middle of a retrieval can effectively contribute to performance improvements, for example, when there are a large number of query sequences.

### Industrial Applicability

As set forth above, according to the present invention, for example, even if an error or a displacement is included in the number of consecutive identical bases in a homopolymer region, due to, for example, the method for determining a base sequence, or the polymorphism of a sequence itself, it is possible to avoid the influence thereof, thereby enabling a more accurate homology retrieval. Moreover, since a homology retrieval can be accurately performed in this way, it is also possible to accurately make a determination, for example, as to whether a query sequence and a partial sequence in a target sequence show only a single homology (similarity) accurately. Furthermore, since compressed sequences, which do not require taking into consideration the number of consecutive identical bases in a homopolymer region, are first compared, and the matched partial sequence of the target sequence is selected, it is also possible to realize cost reductions as compared with conventional technologies due to a further improved data processing capability. Accordingly, the present invention can solve the influence of variations in the number of consecutive identical bases in a homopolymer region, which has been conventionally unsolvable, in the field of homology retrieval (similarity retrieval), and therefore can be considered as a very useful technology particularly in the field of gene analysis.

## Claims

1. A homology retrieval system that retrieves, using sequence information of a query sequence comprising a nucleic-acid base sequence, a partial sequence homologous with the query sequence from sequence information of a genomic-scale target sequence comprising a nucleic-acid base sequence, the system comprising:
an acquisition unit that acquires the sequence information of the query sequence and the target sequence;
a compressed sequence preparation unit that prepares a compressed query sequence and a compressed target sequence in each of which a homopolymer region including two or more consecutive identical bases is replaced with a single base of the bases respectively for the query sequence and the target sequence that have been acquired;
a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
a consecutive base number preparation unit that prepares information on the number of consecutive identical bases in each of the sequences before compression of the compressed query sequence and the compressed candidate sequence selected by the retrieval unit;
a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in the number of consecutive bases;
a selection unit that ranks and selects an arbitrary number of candidate sequences having relatively high homology with the query sequence, based on a degree of similarity computed by the similarity degree computing unit; and
an output unit that outputs information on the arbitrary number of candidate sequences selected by the selection unit.

2. The homology retrieval system according to claim 1,
wherein the acquisition unit comprises an input unit that inputs the sequence information of the query sequence, and a target sequence storage unit in which the sequence information of the target sequence is stored.

3. The homology retrieval system according to claim 1,
wherein the sequence information acquired by the acquisition unit is the compressed query sequence and the compressed target sequence in each of which a homopolymer region including two or more consecutive identical bases is replaced with a single base of the bases.

4. The homology retrieval system according to claim 1,
wherein the compressed sequence preparation unit is a compressing conversion unit that compressingly converts the query sequence and the target sequence that have been acquired respectively into a compressed query sequence and a compressed target sequence in each of which a homopolymer region including two or more consecutive identical bases is replaced with a single base of the bases.

5. The homology retrieval system according to claim 1,
wherein the consecutive base number preparation unit is a counting unit that counts the number of consecutive identical bases in each of the sequences before compression of the compressed query sequence and the compressed candidate sequence selected by the retrieval unit.

6. The homology retrieval system according to claim 1,
wherein the similarity degree computing unit uses a degree of mismatch in the number of consecutive bases for each corresponding base as a penalty score excluding a mismatch where the number of consecutive bases in an upstream terminal base or a downstream terminal base of the compressed query sequence before compression is less than the number of consecutive bases in an upstream terminal base or a downstream terminal base of the compressed candidate sequence before compression, and computes a degree of similarity by adding the penalty scores for each corresponding base.

7. The homology retrieval system according to claim 1, further comprising:
a storage unit in which information on the query sequence and the arbitrary number of candidate sequences selected by the selection unit is stored,
wherein, when a new degree of similarity of a new candidate sequence to the query sequence has been computed by the similarity degree computing unit, the selection unit re-selects, based on the new degree of similarity and the degree of similarity to the query sequence of the arbitrary number of candidate sequences previously stored by the query sequence storage unit, an arbitrary number of candidate sequences from said candidate sequences.

8. The homology retrieval system according to claim 1,
wherein the compressed target sequence is a compressed target partial sequence group in which a homopolymer region including two or more consecutive identical bases is replaced with a single base of the bases for a partial sequence group resulting from dividing the target sequence after compression into fixed lengths.

9. The homology retrieval system according to claim 8,
wherein the retrieval unit is a hash retrieval unit that uses the compressed query sequence and compressed target partial sequences of the compressed target partial sequence group as a key, and performs a refining search for a compressed target partial sequence that matches the compressed query sequence by performing a hash retrieval using the same hash function.

10. The homology retrieval system according to claim 8, further comprising
a target sequence hash table generating unit that uses compressed target partial sequences of the compressed target partial sequence group as a key, and generates a target sequence hash table using the same hash function,
wherein the retrieval unit is a hash retrieval unit that uses the compressed query sequence as a key, and performs a refining search for a compressed target partial sequence that matches the compressed query sequence by performing a hash retrieval with the target sequence hash table generated by the target sequence hash table generating unit, using the same hash function as that used by the target sequence hash table generating unit.

11. The homology retrieval system according to claim 8, further comprising
a query sequence hash table generating unit that uses two or more pieces of the compressed query sequences as a key, and generates a query sequence hash table using the same hash function,
wherein the retrieval unit is a hash retrieval unit that uses compressed target partial sequences of the compressed target partial sequence group as a key, and performs a refining search for compressed target partial sequences that match the compressed query sequences by performing a hash retrieval with the query sequence hash table generated by the query sequence hash table generating unit, using the same hash function as that used by the query sequence hash table generating unit.

12. The homology retrieval system according to claim 11, further comprising
a hash table updating unit that updates data of the query sequence hash table,
wherein, when two or more candidate sequences having the same degree of similarity that show the highest homology are selected for a single query sequence by the selection unit, the hash table updating unit deletes the query sequence and the two or more candidate sequences selected therefor from the data of the query sequence hash table.

13. A homology retrieval system that retrieves, using sequence information of a query sequence comprising a nucleic-acid base sequence, a partial sequence homologous with the query sequence from sequence information of a genomic-scale target sequence comprising a nucleic-acid base sequence, the system comprising:
a terminal and a server,
the terminal and the server being connectable via a communication network outside the system,
the terminal comprising:
a terminal-side transmission unit that transmits information within the terminal to the server via the communication network;
a terminal-side receiving unit that receives information transmitted from the server via the communication network;
a display unit that displays information within the terminal; and
an acquisition unit that acquires the sequence information of the query sequence,
the server comprising:
a server-side transmission unit that transmits information within the server to the terminal via the communication network;
a server-side receiving unit that receives information transmitted from the terminal via the communication network;
a target sequence database in which a target sequence is stored,
a compressed sequence preparation unit that prepares a compressed query sequence and a compressed target sequence in each of which a homopolymer region including two or more consecutive identical bases is replaced with a single base of the bases respectively for the target sequence in the target sequence database and the query sequence received by the server-side receiving unit;
a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
a consecutive base number preparation unit that prepares information on the number of consecutive identical bases in each of the sequences before compression of the compressed query sequence and the compressed candidate sequence selected by the retrieval unit;
a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in the number of consecutive bases; and
a selection unit that ranks and selects an arbitrary number of candidate sequences having relatively high homology with the query sequence, based on a degree of similarity computed by the similarity degree computing unit,
wherein information on the query sequence is transmitted from the terminal-side transmission unit to the server-side receiving unit, information on the arbitrary number of candidate sequences selected by the selection unit of the server is transmitted from the server-side transmission unit to the terminal-side receiving unit, and the information on the arbitrary number of candidate sequences that has been received is displayed by the display unit in the terminal.

14. A server used for the homology retrieval system according to claim 13, the server comprising:
a server-side transmission unit that transmits information within the server to a terminal via the communication network;
a server-side receiving unit that receives information transmitted from the terminal via the communication network;
a target sequence database in which a target sequence is stored,
a compressed sequence preparation unit that prepares a compressed query sequence and a compressed target sequence in each of which a homopolymer region including two or more consecutive identical bases is replaced with a single base of the bases respectively for the target sequence in the target sequence database and the query sequence received by the server-side receiving unit;
a retrieval unit that compares the compressed query sequence and the compressed target sequence, and performs a refining search for a compressed target partial sequence that matches the compressed query sequence in the compressed target sequence, and selects the refined compressed target partial sequence as a compressed sequence of a candidate sequence (compressed candidate sequence);
a consecutive base number preparation unit that prepares information on the number of consecutive identical bases in each of the sequences before compression of the compressed query sequence and the compressed candidate sequence selected by the retrieval unit;
a similarity degree computing unit that compares, based on the information on the number of consecutive identical bases, the number of consecutive bases between the compressed query sequence and the compressed candidate sequence for each corresponding base, and computes a degree of similarity indicating homology of the candidate sequence with the query sequence from a degree of match or a degree of mismatch in the number of consecutive bases; and
a selection unit that ranks and selects an arbitrary number of candidate sequences having relatively high homology with the query sequence, based on a degree of similarity computed by the similarity degree computing unit.

15. A terminal used for the homology retrieval system according to claim 13,
the terminal comprising:
a terminal-side transmission unit that transmits information within the terminal to the server via the communication network;
a terminal-side receiving unit that receives information transmitted from the server via the communication network;
a display unit that displays information within the terminal; and
an acquisition unit that acquires sequence information of the query sequence,
wherein information on the query sequence is transmitted from the terminal-side transmission unit to the server-side receiving unit, information on the arbitrary number of candidate sequences selected by the selection unit of the server is transmitted from the server-side transmission unit to the terminal-side receiving unit, and the information on the arbitrary number of candidate sequences that has been received is displayed by the display unit in the terminal.

16. A homology retrieval apparatus that retrieves, using sequence information of a query sequence comprising a nucleic-acid base sequence, a partial sequence homologous with the query sequence from sequence information of a genomic-scale target sequence comprising a nucleic-acid base sequence, the apparatus comprising the homology retrieval system according to claim 1.

17. A homology retrieval method for retrieving, using sequence information of a query sequence comprising a nucleic-acid base sequence, a partial sequence homologous with the query sequence from sequence information of a genomic-scale target sequence comprising a nucleic-acid base sequence, the method comprising:
an acquisition step of acquiring the sequence information of the query sequence and the target sequence;
a compressed sequence preparation step of preparing a compressed query sequence and a compressed target sequence in each of which a homopolymer region including two or more consecutive identical bases is replaced with a single base of the bases respectively for the query sequence and the target sequence that have been acquired;
a retrieval step of comparing the compressed query sequence and the compressed target sequence, and performing a refining search for a compressed target partial sequence that matches the compressed query sequence in the compressed target sequence, and selecting the refined compressed target partial sequence as a compressed sequence of a candidate sequence (compressed candidate sequence);
a consecutive base number preparation step of preparing information on the number of consecutive identical bases in each of the sequences before compression of the compressed query sequence and the compressed candidate sequence selected in the retrieval step;
a similarity degree computing step of comparing, based on the information on the number of consecutive identical bases, the number of consecutive bases between the compressed query sequence and the compressed candidate sequence for each corresponding base, and computing a degree of similarity indicating homology of the candidate sequence with the query sequence from a degree of match or a degree of mismatch in the number of consecutive bases;
a selection step of ranking and selecting an arbitrary number of candidate sequences having relatively high homology with the query sequence, based on a degree of similarity computed in the similarity degree computing step; and
an output step of outputting information on the arbitrary number of candidate sequences selected by the selection step.

18. The homology retrieval method according to claim 17,
wherein the acquisition step comprises an input step of inputting the query sequence, and a calling step of calling sequence information of the target sequence from the target sequence that has been stored in a target sequence storage step.

19. The homology retrieval method according to claim 17,
wherein the similarity degree computing step uses a degree of mismatch in the number of consecutive bases for each corresponding base as a penalty score excluding a mismatch where the number of consecutive bases in an upstream terminal base or a downstream terminal base of the compressed query sequence before compression is less than the number of consecutive bases in an upstream terminal base or a downstream terminal base of the compressed candidate sequence before compression, and computes a degree of similarity by adding the penalty scores for each corresponding base.

20. The homology retrieval method according to claim 17,
wherein, when a new degree of similarity of a new candidate sequence to the query sequence has been computed in the similarity degree computing step, the selection step re-selects, based on the new degree of similarity and the degree of similarity to the query sequence of the arbitrary number of candidate sequences previously selected by the selection step, an arbitrary number of candidate sequences from said candidate sequences.

21. A computer program capable of executing the homology retrieval method according to claim 17 on a computer.

22. An electronic medium in which the computer program according to claim 21 is stored.
